# EUROPÄISCHE PATENTSCHRIFT

(11) **EP 3 553 160 B1**
(45) Veröffentlichungstag und Bekanntmachung des Hinweises auf die Patenterteilung: **09.06.2021**
(21) Anmeldenummer: 19167879.6
(22) Anmeldetag: 08.04.2019
(51) Int. Cl.: C12M 1/00, C12M 3/00, C12M 1/12, C12M 1/34

(54) **SENSORAUFNAHME FÜR EINEN BIOREAKTOR SOWIE BIOREAKTOR MIT SENSORAUFNAHME UND VERFAHREN ZUR VERMEHRUNG ODER KULTIVIERUNG BIOLOGISCHEN MATERIALS**
SENSOR HOLDER FOR A BIOREACTOR AND BIOREACTOR WITH SENSOR HOLDER AND METHOD FOR AUGMENTING OR CULTURING BIOLOGICAL MATERIAL
LOGEMENT DE CAPTEUR POUR UN BIORÉACTEUR AINSI QUE BIORÉACTEUR POURVU DE LOGEMENT DE CAPTEUR ET PROCÉDÉ DE REPRODUCTION OU DE CULTURE DE LA MATIÈRE BIOLOGIQUE

(30) Priorität: 09.04.2018 DE 102018108325
(43) Veröffentlichungstag der Anmeldung: 16.10.2019
(60) Teilanmeldung: 21157462.9
(73) Patentinhaber: SCHOTT AG, 55122 Mainz (DE)
(72) Erfinder: OTT, Christian, 84539 Ampfing (DE); HETTLER, Robert, 84036 Kumhausen (DE)
(74) Vertreter: Blumbach · Zinngrebe Patentanwälte PartG mbB

(56) Entgegenhaltungen:
- WO-A1-2018/014984
- DE-A1-102008 036 934
- DE-A1-102009 037 345
- DE-A1-102010 007 559
- DE-A1-102010 012 162
- DE-A1-102013 015 106
- DE-U1- 8 703 478

## Beschreibung

Die Erfindung betrifft eine Sensoraufnahme für einen Bioreaktor sowie einen Bioreaktor mit einer Sensoraufnahme und Verfahren zur Vermehrung oder Kultivierung biologischen Materials.

Verfahren zur Herstellung biologischen Materials, wie beispielsweise biotechnologische Produktionsprozesse, welche die Kultivierung von Mikroorganismen, tierischen und pflanzlichen Zellen umfassen sind von zunehmender Bedeutung. Biotechnologische Produktionsprozesse umfassen beispielsweise die Kultivierung von Mikroorganismen, tierischen und pflanzlichen Zellen.

Herkömmlich werden Messsonden vor der Sterilisation des Bioreaktors installiert, sowohl bei Einwegbioreaktoren, welche auch als Single-Use-Reaktoren bezeichnet werden, als auch bei für die Mehrfachnutzung vorgesehenen Bioreaktoren, die fachüblich auch Multi-Use-Reaktoren genannt werden. Derartige Messsonden, wie beispielsweise pH-Sonden, werden generell vor dem Sterilisieren kalibriert.

Ein Wechsel von Messeinrichtungen zur Erfassung verschiedener physikalischer, chemischer oder biologischer Messgrößen während des Produktionsprozesses, insbesondere der Kultivierung, ist herkömmlich mit hohen Fertigungsrisiken, bis hin zum vollständigen Ausfall einer Charge verbunden. Ein Fehler beim Autoklavieren, oder währen der Kultivierung impliziert daher in der Regel auch einen Prozesskontrollverlust.

Die Ermittlung von Substrat- und Produktkonzentrationen ist herkömmlich sehr zeitaufwändig. Bedingt ist dies durch den Probenzug, mit dem regelmäßig auch ein Kontaminationsrisiko verbunden ist, sowie durch eine ressourcenintensive Off-Line-Analytik.

DE 10 2010 063 031 A1 zeigt einen potentiometrischen Sensor sowie ein Verfahren zur Inbetriebnahme eines potentiometrischen Sensors. Um den Einsatz eines als Einweg-Fermenter oder Einweg-Bioreaktor dienenden Behälters besonders einfach zu gestalten, kann die dort offenbarte potentiometrische Sonde bereits vor der Sterilisation, beispielsweise durch Bestrahlung mit Gammastrahlung, über einen Anschluss fest in eine Wand des Behälters eingebaut werden und für die Dauer der Lagerung und Verwendung darin verbleiben.

In dem Dokument DE 10 2006 022 307 A1 wird ein Einwegbioreaktor mit reversibel, äußerlich anbringbarer Sensoranordnung zur Messung einer physikalischen Größe eines enthaltenen Mediums beschrieben, wobei in wenigstens einer dem Zu- und/oder Abfluss von Medium dienenden Peripherieleitung des Bioreaktors ein Sensoradapter zur Aufnahme einer über eine innere Grenzfläche des Sensoradapters mit die Peripherieleitung durchströmendem Medium wechselwirkenden, elektronischen Sensoranordnung integriert ist. Da die Messung jeweils nur in der Peripherieleitung des Bioreaktors vorgenommen werden kann, ist eine Prozesskontrolle innerhalb des Bioreaktors mit dieser Anordnung nicht möglich.

DE 10 2010 037 923 A1 offenbart eine Bioreaktor-Anordnung für Zellen enthaltend einen abgeschlossenen Bioreaktor, einen Zellpelletträger zur Aufnahme eines Zellpellets und Mittel zum Zuführen von Nährlösung in das Zellpellet. Diese Anordnung erlaubt die kontaktlose Messung des Sauerstoffgehalts. Mit einem Laser werden hierbei Sauerstoffsonden zur Phosphoreszenz angeregt. Das emittierte Phosphoreszenzsignal wird mit dem Detektor aufgenommen und an eine Auswerteelektronik geleitet. Hierzu weist der Bioreaktor lichtdurchlässige Fenster auf und der Laser und der Detektor sind außerhalb des Bioreaktors angeordnet. Ein Austausch von Sensoren oder Messeinrichtungen wird in diesem Dokument nicht beschrieben.

DE 10 2008 036934 offenbart einen Bioreaktor mit einer Durchführung (Sonden-/Sensoraufnahme) mit einer Durchführung (Sensoraufnahme) mit einer Durchgangsöffnung, die mit einem transparenten Fenster hermetisch abgeschlossen ist für das Einführen eines Sensors.

DE 10 2011 101 108 A1 beschreibt eine Transflexionssonde zur Durchführung einer Transflexionsmessung an einem in einem starren Behälter befindlichen Fluid mit einen mit einer Lichtleitstrecke in seinem Inneren ausgestatteten Sondenschaft, an dessen vorderer Stirnseite eine offene Durchflusskammer mit einer der vorderen Stirnseite des Sondenschaftes gegenüberliegenden Reflexionsscheibe angeordnet ist. Der Sondenschaft ist als ein an seiner vorderen Stirnseite von einem transparenten Fenster abgeschlossener, starrer Hohlkörper ausgebildet ist, der an seinem rückwärtigen Ende eine erste Koppeleinrichtung zur starren Ankopplung eines Sensormoduls an den Sondenschaft aufweist. Diese Koppeleinrichtung ist jedoch mit der offenen Durchflusskammer und insbesondere mit der der vorderen Stirnseite des Sondenschaftes gegenüberliegenden Reflexionsscheibe fest verbunden, sodass der Austausch von Sensormodulen an einer Sonde beziehungsweise die wechselnde Ankopplung eines Sensormoduls an unterschiedliche Sonden desselben oder verschiedenen Behälter ermöglicht wird. Ein Austausch von Sensoren zur Messung physikalischer, chemischer oder biologischer Messgrößen wird nicht offenbart.

Insbesondere für Single-Use- oder Einmal-Anwendungen steht kein View-Port (Sichtöffnung des Bioreaktors) zur Verfügung, der die optischen Anforderungen für eine geeignete Messtechnik insbesondere die Anforderungen an Transmission und Distorsionsfreiheit erfüllt und den Sensor, oder die Sonde dabei austauschbar und mechanisch zuverlässig trägt.

Nach dem Stand der Technik erfüllt derzeit kein System auf dem Markt die Anforderung, zuverlässig den sterilen Bereich der Kultivierung und den der Messung hermetisch voneinander zu trennen und dabei die Verwendung von Sensoren für unterschiedliche Messverfahren zu ermöglichen.

Der Erfindung liegt die Aufgabe zugrunde, eine auch als Sensorport bezeichnete Sensoraufnahme sowie einen hiermit versehenen Bioreaktor bereitzustellen, mit welchen jeweils ein flexibler Austausch verschiedener Sensoren ermöglicht wird, ohne dass hierbei ein Zugang zum Inneren des Bioreaktors eröffnet wird.

Diese Aufgabe wird durch den Gegenstand der unabhängigen Ansprüche gelöst. Vorteilhafte Weiterbildungen sind den abhängigen Ansprüchen sowie der Beschreibung zu entnehmen.

Ein erfindungsgemäßer Bioreaktor umfasst einen Behälter zur Aufnahme von fluiden Medien, die biologisches Material enthalten, eine Durchführung mit einer Durchgangsöffnung zwischen dem Inneren des Behälters zur Aufnahme von fluiden Medien, die biologisches Material enthalten, und dem Äußeren des Behälters zur Aufnahme von fluiden Medien, die biologisches Material enthalten, und es erstreckt sich eine Sensoraufnahme zum Halten von zumindest einem Sensor zumindest teilweise in der Durchgangsöffnung der Durchführung.

Bevorzugt umfasst die Durchführung ist einen Standardport, wie beispielsweise einen Ingold-Port, einen Broadly-James-Port, einen B.-Braun-Sicherheitsport oder einen einem andern Standard entsprechenden Port. Diese weisen jeweils eine Öffnung definierten Durchmessers auf, welche in typischer Weise das Innere eines Bioreaktors mit dessen Äußerem verbindet oder eröffnet.

Generell kann der Behälter zur Aufnahme von fluiden Medien, die biologisches Material enthalten, der Behälter eines Multi-Use-Bioreaktors zur Mehrfachanwendung sein.

Vorteilhaft umfasst der Behälter zur Aufnahme von fluiden Medien, die biologisches Material enthalten, dann Edelstahl oder besteht dieser aus Edelstahl.

Alternativ kann der Behälter zur Aufnahme von fluiden Medien, die biologisches Material enthalten, auch der Behälter eines Single-Use-Bioreaktors zur Einweganwendung sein.

Vorteilhaft ist es in diesem Fall, wenn der Behälter zur Aufnahme von fluiden Medien, die biologisches Material enthalten, einen Kunststoff, insbesondere einen sterilisierbaren Kunststoff umfasst oder aus einem Kunststoff, insbesondere einem sterilisierbaren Kunststoff besteht. Dieser Kunststoff kann ein polymeres Material umfassen und kann insbesondere aus einem geeigneten Material bestehen, welches eine Gamma- bzw. eine chemische Sterilisierung mit ETO übersteht.

Als Kunststoffe sind beispielsweise Polyester Elastomere mit EVOH (Ethylen-Vinyllalkohol-Copolymer) oder Polyethylene geeignet. Hierbei kann eine Materialmischung verwendet werden, bei welcher beispielsweise ein Schichtsystem eine Außenschicht aufweist, welche mechanische Stabilität bereitstellt. Eine gasdichte Zwischenschicht kann sich dann an eine innenliegende biokompatible Schicht anschließen.

Um den strengen Anforderungen für die Herstellung von Biopharmazeutika zu genügen, kann das Material, welches der Bioreaktor und welches die Sensoraufnahme jeweils umfassen so ausgewählt sein, dass dieses jeweils den nachfolgenden Standards entspricht:
i) FDA approved materials (ICH Q7A, CFR 211.65(a) - Code of Federal Regulations, USP Class, animal derivative free, bisphenol A free)
ii) EMA (European Medicines Agency) EU GMP Guide Part II approved materials
iii) Sectoral chemical resistance - ASTM D 543-06
iv) Biocompatibility e.g. referred to US Pharmacopeia or tests referred to ISO 10993.

Um messtechnisch günstige Verhältnisse und eine mechanisch stabile Halterung der Sensoraufnahme und insbesondere einer in der Sensoraufnahme gehaltenen sensorischen Einrichtung zu erlangen, kann sich die Sensoraufnahme innerhalb der Durchführung, insbesondere deren Durchgangsöffnung formschlüssig zur Durchgangsöffnung der Durchführung erstreckt, wobei die Sensoraufnahme mit deren Fenster die Durchgangsöffnung verschließt.

Die Erfindung umfasst ferner eine Sensoraufnahme zum Halten von zumindest einem Sensor für einen Bioreaktor, insbesondere für einen Bioreaktor, wie dieser vorstehen beschrieben wurde, welche einen Halterungskörper mit einem Sensoraufnahmebereich umfasst, an dem ein Fenster mit einem transparenten Element angeordnet ist, das für elektromagnetische Strahlung transmittierend ist. Hierdurch lassen sich optische Signale oder Messgrößen mit verschiedenen sensorischen Einrichtungen erfassen, die auch während einer Kultivierung austauschbar gehalten sind, wobei das Risiko einer Kontaminierung erheblich vermindert ist. Transmittierend wird hierbei als Oberbegriff für transparent und lichtdurchlässig, wie beispielsweise diffus streuend lichtdurchlässig verwendet. So wird beispielsweise eine optische Mattscheibe, welche an zumindest einer von deren Oberflächen diffus streuend ausbebildet ist auch als transmittierend bezeichnet.

Besonders vorteilhaft ist es, wenn der Bioreaktor zusammen mit der Sensoraufnahme zum Halten von zumindest einem Sensor autoklavierbar ist, insbesondere während diese zumindest teilweise in der Durchgangsöffnung der Durchführung gehalten ist, autoklavierbar ist, denn dann kann hierdurch eine Kontamination mit biologisch aktivem oder wechselwirkendem Material mit sehr hoher Sicherheit ausgeschlossen werden.

Hierzu ist insbesondere auch die Sensoraufnahme zum Halten von zumindest einem Sensor autoklavierbar ausgebildet.

Überraschend hat es sich gezeigt, dass bei den hier offenbarten Sensoraufnahmen zum Halten von zumindest einem Sensor 3500 Auktoklavierungs-Zyklen bei 2 bar und 134°C möglich waren.

Autoklavierbar wird im Rahmen dieser Offenbarung auch als autoklavierbar im Sinne der DIN EN ISO 14937; EN ISO 17665, welche für Medizinprodukte gültig ist, verstanden.

Vorteilhaft ist der Halterungskörper zylindersymmetrisch, insbesondere säulenförmig ausgebildet und weist eine Durchgangsöffnung auf, die auf der dem Inneren des Bioreaktors zugeordneten Seite mittels des Fensters fluiddicht, insbesondere hermetisch dicht abgeschlossen ist. Auch die Hermetizität sichert weiter einen kontaminationsfreien Betrieb des jeweiligen Bioreaktors.

Bevorzugt weist das transparente Element des Fensters in einem Spektralbereich mit einer Wellenlänge von 250 bis 2000 nm eine Transmission auf, die höher ist als 80%, besonders bevorzugt höher als 90 %.

Bei den bevorzugten Ausführungsformen kann das transparente Element des Fenster Glas umfassen oder aus Glas bestehen.

Hierbei umfasst das Glas des transparenten Elements des Fensters Quarzglas oder Borosilikatglas umfasst oder besteht aus diesem.

Wenn das transparente Element des Fensters mittels einer GTMS-Druckverglasung an einem Grundkörper und dieser am Halterungskörper der Sensoraufnahme gehalten ist, wird eine mechanisch sowie thermisch stabile Verbindung bereitgestellt, welche auch gegenüber Autoklavierung sowie einer Reinigung und Sterilisierung mittels der als CIP (Clean-In-Place) und als SIP (Sterilization-In-Place) bezeichneten Verfahren dauerhaft und betriebssicher beständig ist.

Besonders bevorzugt ist dabei das Fenster mittels Schweißen, insbesondere Laser-Schweißen hermetisch dicht mit dem Halterungskörper verbunden.

Bei einer besonders bevorzugten Ausführungsform ist das transparente Element des Fensters scheibenförmig ausgebildet und weist insbesondere planparallele Hauptoberflächen auf.

Bei weiteren bevorzugten Ausführungsformen ist das transparente Element des Fensters plankonvex, plankonkav, bikonvex, bikonkav, konvexkonkav oder konkavkonvex geformt. Besonders in diesem letztgenannten Fall kann das transparente Element des Fensters auch ein Teil eines diesem zugeordneten bildgebenden Systems sein.

Der Halterungskörper kann einen seitlichen, sich in radialer Richtung erstreckenden Absatz aufweisen, um insbesondere damit einen Anschlag in axialer Richtung an einem am Bioreaktor angeordneten Standardport auszubilden.

Dann kann bei in die Durchgangsöffnung eingesetzter Sensoraufnahme der axiale Abstand des transparenten Elements des Fensters zur Innenwand des Bioreaktors durch den axialen Abstand des transparenten Elements des Fensters zum seitlichen Absatz definiert werden. Dann ist das transparente Element des Fensters bei in die Durchgangsöffnung eingesetztem Halterungskörper der Sensoraufnahme, insbesondere bei Anlage des seitlichen Absatzes an einem Standardport vorteilhaft innerhalb des Bioreaktors angeordnet. Ein Satz von Halterungskörpern mit verschiedenem axialem Abstand des Fensters, insbesondere des transparenten Elements des Fensters zum seitlichen Absatz kann hierbei eine wählbare axiale Positionierung des Fensters innerhalb des Bioreaktors ermöglichen. Als axialer Abstand wird im Rahmen dieser Offenbarung ein Abstand verstanden, der in Richtung der Symmetrieachse des zylindersymmetrischen Halterungskörpers der Sensoraufnahme jeweils gemessen oder angegeben ist.

Der Halterungskörper umfasst erfindungsgemäß einen Standardflansch, welcher sich vorzugsweise radial nach außen erstreckt und in axialer Richtung eine Anlagefläche für ein Dichtmittel definiert. Diese Anlagefläche kann beispielsweise eine Anlagefläche sein, wie diese beispielsweise bei einem Ingold-Port, einen Broadly-James- Port, einen B.-Braun-Sicherheitsport oder einen einem andern Standard entsprechenden Port verwendet wird.

Der Sensoraufnahme kann eine Abdeckkappe mit einer Anlagefläche für ein Dichtmittel zugeordnet sein, welche beispielsweise Teil eines Sensors ist.

Vorteilhaft bilden dabei der Flansch des Halterungskörpers und ein Flansch der Abdeckkappe jeweils einen zumindest bereichsweise schräg verlaufenden Abschnitt einer Ringschulter aus.

Ein der Sensoraufnahme zugeordneter kann zumindest einen ersten Sensorabschnitt aufweisen, welcher in die Durchgangsöffnung des Halterungskörpers vorzugsweise formschlüssig einbringbar ist und zumindest eine sensorische Einrichtung umfassen.

Bei bevorzugten Ausführungsformen umfasst bei einem Sensor für die hier beschriebene Sensoraufnahme die sensorische Einrichtung einen Bildleiter, insbesondere einen Faser-Bildleiter.

Die sensorische Einrichtung kann aber auch ein bildgebendes optisches System, insbesondere zusammen mit einer bildaufnehmenden Einrichtung umfassen. Dies kann beispielsweise ein bildaufnehmender Sensor einer digitalen Aufzeichnungsvorrichtung sein, welcher zur Dokumentation oder zum Real-Time-Monitoring einsetzbar ist.

Wenn die sensorische Einrichtung ein Spektrometer umfasst, können hiermit beispielsweise photolumineszente Messungen durchgeführt werden, welche die Separierung des Lichts eines die Photoemission anregenden Lasers von dem photolumineszent emittierten Licht gestattet.

Essentiell für die Produktausbeute sind jedoch generell, neben Parametern wie der Temperatur, welche auch spektral erfassbar ist, das Monitoring oder die messtechnische Überwachung von metabolismus- und produktbildungsrelevanten Stoffen in Echtzeit.

Ein bevorzugtes Verfahren zur Vermehrung oder Kultivierung biologischen Materials umfasst das Einbringen fluiden, insbesondere biologischen Materials oder einer Vorstufe biologischen Materials in einen Bioreaktor, wie dieser hier beschrieben ist, sowie das Erfassen einer physikalischen, chemischen oder biologischen Messgröße unter Verwendung einer Sensoraufnahme wie diese hier beschrieben ist sowie eines hier beschriebenen Sensors.

Ein weiteres bevorzugtes Verfahren zur Vermehrung oder Kultivierung biologischen Materials umfasst das Einbringen von fluiden Medien, die biologisches Material oder eine Vorstufe biologischen Materials enthalten in einen Bioreaktor, wie dieser vorliegend offenbart wird, insbesondere in einen Behälter des Bioreaktors zur Aufnahme von fluiden Medien, die biologisches Material enthalten, wobei der Behälter zur Aufnahme von fluiden Medien, die biologisches Material enthalten, eine Durchführung mit einer Durchgangsöffnung zwischen dem Inneren des Behälters zur Aufnahme von fluiden Medien, die biologisches Material enthalten, und dem Äußeren des Behälters zur Aufnahme von fluiden Medien, die biologisches Material enthalten aufweist, und eine Sensoraufnahme, welche ein Fenster mit einem transparenten Element, das für elektromagnetische Strahlung transparent ist, an oder in der Durchführung des Behälters zur Aufnahme von fluiden Medien, die biologisches Material enthalten, vor dem Einbringen der fluiden Medien, die biologisches Material oder eine Vorstufe biologischen Materials enthalten, angebracht wird.

Mit großem Vorteil können die hier offenbarten Verfahren zur Vermehrung oder Kultivierung die Herstellung von Pharmazeutika, insbesondere von Biopharmazeutika umfassen.

Bevorzugt wird der Bioreaktor nach dem Anbringen der Sensoraufnahme sterilisiert.

Verfahrensgemäß wird der Bioreaktor nach Anbringen der Sensoraufnahme, mit einem Sensor bestückt, wobei der Sensor zumindest abschnittsweise in den Sensoraufnahmebereich des Halterungskörpers eingebracht wird..

Es kann dann das Erfassen von sensorischen Messwerten eines Sensors erfolgen, welcher in einer hier offenbarten Sensoraufnahme angeordnet ist und es können auch während der Kultivierung oder Vermehrung Sensoren ausgetauscht und insbesondere auch verschiedenartige Sensoren eingesetzt werden.

Beispiele hierfür sind Mikroskopsonden, generell Sensoren, welch elektromagnetische Strahlung erfassen können, beispielsweise im Ultravioletten oder auch im ultravioletten und sichtbaren Spektralbereich sowie Infrarot-Sensoren. Ferner können die Sensoren Einrichtungen zur Trübungsmessung sowie für die Ramanspektroskopie zur Messung der dielektrischen Verschiebung umfassen. Bei letztgenannten Sensor kann der Sensor auch eine Anregungslichtquelle für die Ramanspektroskopie umfassen.

Ferner kann auch ein Sensor zur Messung der Polarisationsänderung von Emissionslicht, welches vom biologischen Material abgegeben wurde, umfassen.

Vorteilhaft kann die Sensoraufnahme zumindest einen Sensor aufnehmen, und im Betriebszustand die Strahlungsintensität und/oder die Wellenlänge der elektromagnetischen Strahlung im Innern des Bioreaktors, insbesondere im Innern des Behälters zur Aufnahme fluider Medien, welche biologisches Material enthalten, messen.

Wenn die Sensoraufnahme zumindest einen Sensor aufnimmt, und im Betriebszustand die Strahlungsintensität und/oder die Wellenlänge der elektromagnetischen Strahlung im Innern des Bioreaktors, insbesondere im Innern des Behälters zur Aufnahme fluider Medien, welche biologisches Material enthalten, ortsaufgelöst gemessen wird, können beispielsweise Stoffwechselvorgängen ortsaufgelöst erfasst und spezifische beeinflusst werden.

Wenn für einen definierten Zeitraum elektromagnetische Strahlung einer definierten Wellenlänge, bevorzugt 250 nm in den Bioreaktor eingestrahlt wird und nach dieser Einstrahlung breitbandig oder selektiv bei einer Wellenlänge, insbesondere bei 270 nm eine Strahlungsintensität und/oder Wellenlänge der elektromagnetischen Strahlung im Innern des Bioreaktors gemessen wird, können hierdurch fluoreszent emittierte Anteile des Lichts detektiert und bezüglich definierter Stoffwechselvorgänge innerhalb des Photobioreaktors ausgewertet werden.

Vorteilhaft kann mit den hier beschriebenen Vorrichtungen und Verfahren beispielsweise während der Kultivierung biologischen Materials im Behälter des Bioreaktors ein Sensor gewechselt werden, insbesondere ohne, dass sich die Sterilitätsbedingungen innerhalb des Behälters des Bioreaktors während eines Austauschs oder Wechsel des Sensors ändern, somit also aufrecht erhalten werden.

Vorteilhaft kann bei einem Verfahren einer bevorzugten Ausführungsform der Bioreaktor mit dessen Behälter zur Aufnahme von fluiden Medien, die biologisches Material enthalten, und Sensoraufnahme zum Halten von zumindest einem Sensor autoklaviert werden während die Sensoraufnahme zum Halten von zumindest einem Sensor zumindest teilweise in der Durchgangsöffnung der Durchführung gehalten ist, denn dann können Kontaminationen des Photobioreaktors mit sehr viel höherer Wahrscheinlichkeit als bisher vermieden werden und kann die Autoklavierung mit einem einzigen Vorgang durchgeführt werden. Wird folglich die Autoklavierung zeitnahe vor der Befüllung des Photobioreaktors mit dem biologischen Material vorgenommen, reduziert sich auch das Risiko des zwischenzeitlichen Eintrags von Verschmutzungen.

Die Veröffentlichung "Optical Sensor Systems for Bioprocess Monitoring", Stefan Marose, Carsten Lindemann, Roland Ulber and Thomas Scheper, TIBTECH JANUARY 1999 (VOL 17), lehrt, dass die Messung optischer Dichte das universellste Instrument zur in-situ-Massenkontrolle in einem Bioreaktor ist. Mit der vorliegenden Erfindung werden Nachteile derartiger Einrichtungen, insbesondere bezüglich der Prozesssicherheit jedoch stark vermindert.

Bei den hier offenbarten Verfahren können durch Mutagenese veränderte photo- oder mixotrophen Mikroorganismen, insbesondere auch Mikroalgen, Hefen und Bakterien verwendet werden.

Die Erfindung wird nachfolgend anhand bevorzugter Ausführungsformen und unter Bezugnahme auf die beigeschlossenen Zeichnungen detaillierter beschrieben.

Es zeigen:

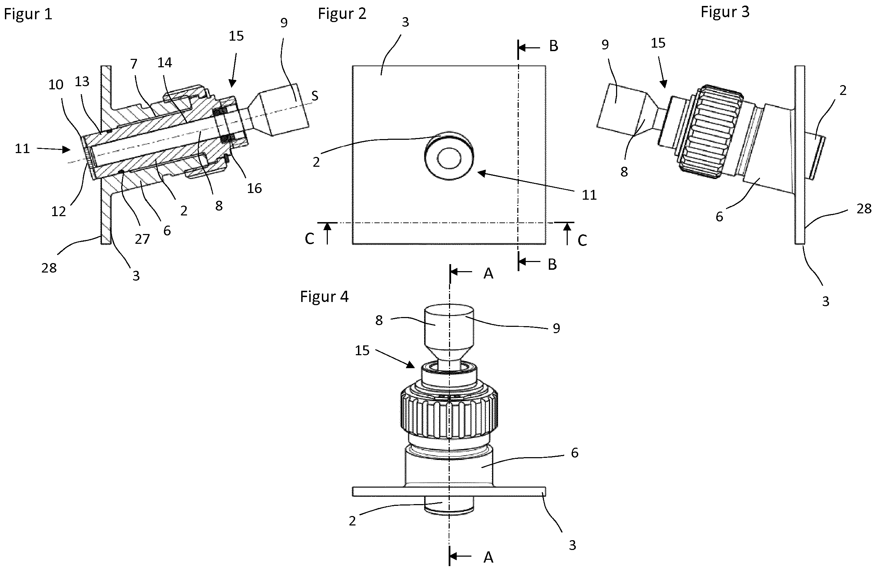

- Figur 1: eine Querschnittsansicht, einer ersten bevorzugten Ausführungsform einer Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 4 dargestellten Schnittebene AA verläuft,
- Figur 2: eine Aufsicht auf die erste bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, schräg von oben, jedoch von der Innenseite des Behälters zur Aufnahme von fluiden Medien aus gesehen,
- Figur 3: eine Querschnittsansicht, der ersten bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 2 dargestellten Schnittebene BB verläuft,
- Figur 4: eine Querschnittsansicht, der ersten bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 2 dargestellten Schnittebene CC verläuft,

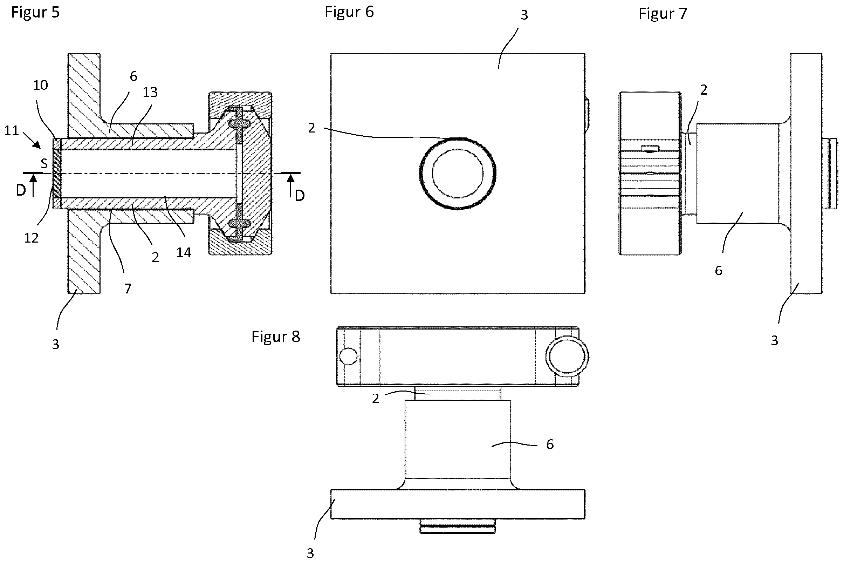

- Figur 5: eine Querschnittsansicht, einer zweiten bevorzugten Ausführungsform einer Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene im Wesentlichen wie bei Figur 1 für die erste Ausführungsform verläuft,
- Figur 6: eine Aufsicht auf die zweite bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, jedoch von der Innenseite des Behälters zur Aufnahme von fluiden Medien aus gesehen,
- Figur 7: eine Querschnittsansicht, der zweiten bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene vertikal durch den Behälter zur Aufnahme biologischen Materials vor der Sensoraufnahme und vor der Durchführung verläuft,
- Figur 8: eine Querschnittsansicht, der zweiten bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene horizontal durch den Behälter zur Aufnahme biologischen Materials vor der Sensoraufnahme und vor der Durchführung verläuft,

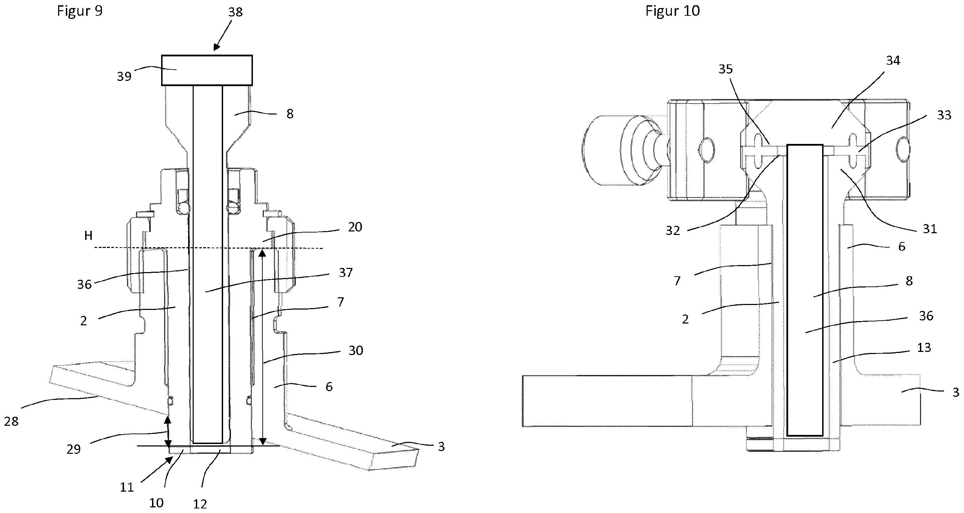

- Figur 9: eine perspektivische Querschnittsansicht entlang der Schnittebene AA aus Figur 4 der ersten bevorzugten Ausführungsform einer Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist,
- Figur 10: eine perspektivische Querschnittsansicht der zweiten bevorzugten Ausführungsform einer Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, entlang der Schnittebene DD aus Figur 5,

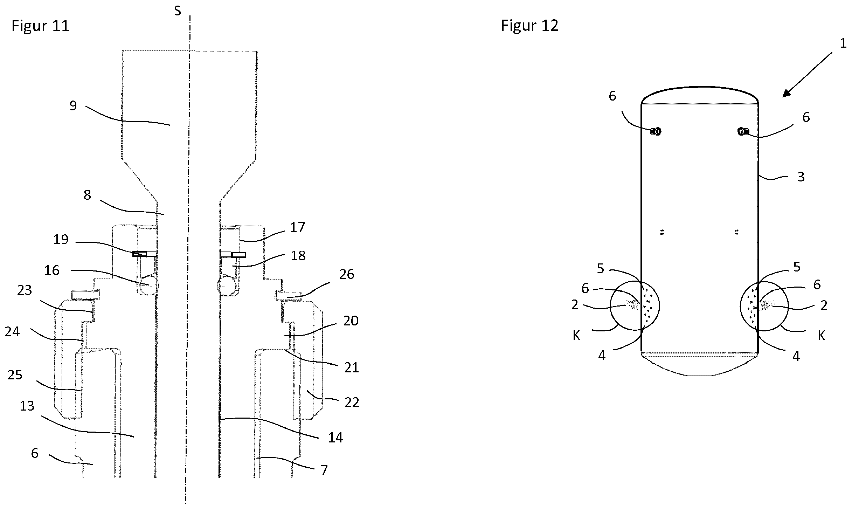

- Figur 11: einen Ausschnitt aus der Darstellung der Figur 9, deren oberen Bereich erfassend,
- Figur 12: eine seitliche Ansicht eines Bioreaktors mit einer Sensoraufnahme zum Halten von zumindest einem Sensor, bei welcher der Behälter zur Aufnahme von fluiden Medien, die biologisches Material enthalten teilweise aufgebrochen dargestellt ist,

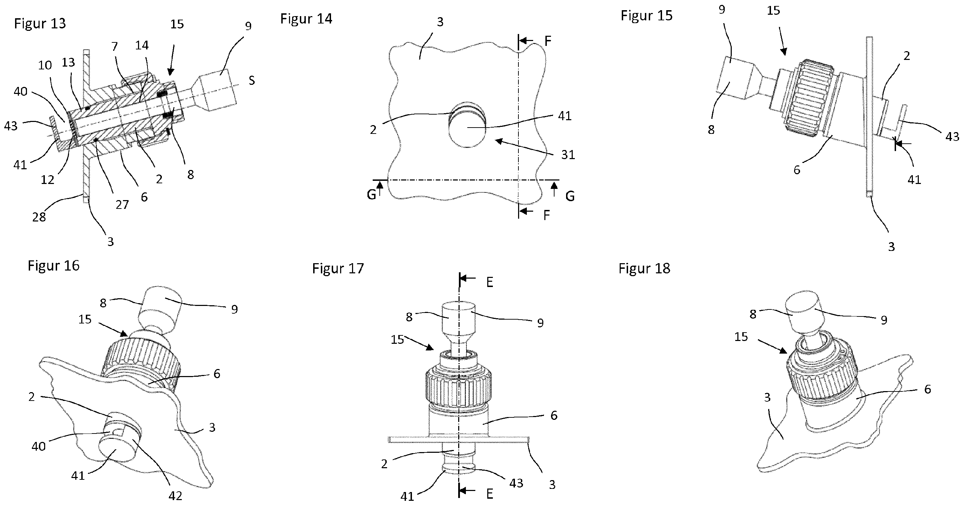

- Figur 13: eine Querschnittsansicht, einer weiteren bevorzugten Ausführungsform einer Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 17 dargestellten Schnittebene EE verläuft, und der Bioreaktor ein Multi-Use-Bioreaktor ist, und bei welcher die Sensoraufnahme zum Halten von zumindest einem Sensor eine Messkammer, insbesondere für Trübungsmessungen aufweist,
- Figur 14: eine Aufsicht auf die weitere bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, schräg von oben, jedoch von der Innenseite des Behälters zur Aufnahme von fluiden Medien aus gesehen,
- Figur 15: eine Querschnittsansicht, der weiteren bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 14 dargestellten Schnittebene FF verläuft,
- Figur 16: eine weitere Aufsicht auf die weitere bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, schräg von seitlich unten, jedoch von der Innenseite des Behälters zur Aufnahme von fluiden Medien aus gesehen,
- Figur 17: eine Querschnittsansicht, der weiteren bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 14 dargestellten Schnittebene GG verläuft,
- Figur 18: eine weitere Aufsicht auf die weitere bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, schräg von seitlich oben, jedoch von der Außenseite des Behälters zur Aufnahme von fluiden Medien aus gesehen,

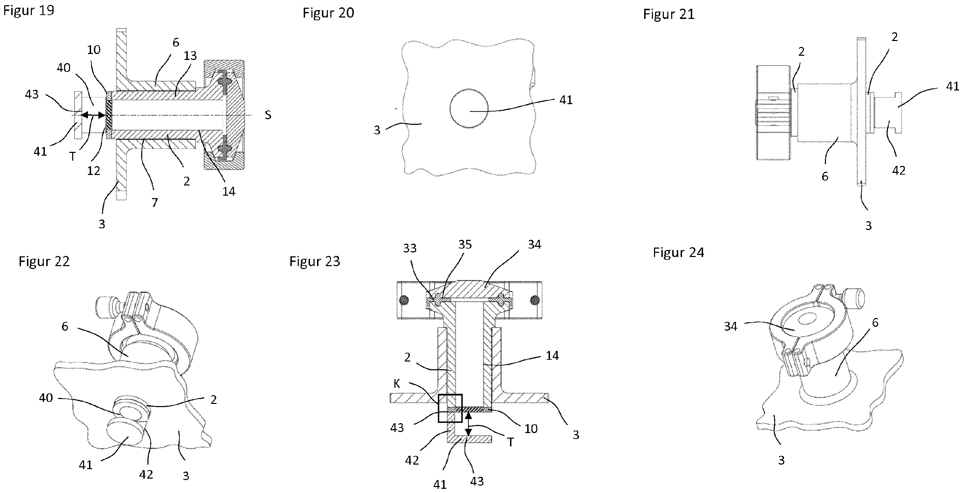

- Figur 19: eine Querschnittsansicht, einer nochmals weiteren bevorzugten Ausführungsform einer Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene im Wesentlichen wie bei Figur 5 dargestellt verläuft, und der Bioreaktor ein Single-Use-Bioreaktor ist, und bei welcher die Sensoraufnahme zum Halten von zumindest einem Sensor eine Messkammer, insbesondere für Trübungsmessungen aufweist,
- Figur 20: eine Aufsicht auf die nochmals weitere bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist von der Innenseite des Behälters zur Aufnahme von fluiden Medien aus gesehen,
- Figur 21: eine Querschnittsansicht, der nochmals weiteren bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene wie in Figur 7 verläuft,
- Figur 22: eine weitere Aufsicht auf die nochmals weitere bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, schräg von oben, jedoch von der Innenseite des Behälters zur Aufnahme von fluiden Medien aus gesehen,
- Figur 23: eine Querschnittsansicht, der nochmals weiteren bevorzugten Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, wobei die Schnittebene wie in Figur 10 verläuft,
- Figur 24: eine weitere Aufsicht auf die nochmals weitere bevorzugte Ausführungsform der Sensoraufnahme zum Halten von zumindest einem Sensor, welche in einer Durchgangsöffnung eines Behälters zur Aufnahme von fluiden Medien, die biologisches Material enthalten, der jedoch nur zum Teil dargestellt ist, angeordnet ist, schräg von seitlich unten, jedoch von der Außenseite des Behälters zur Aufnahme von fluiden Medien aus gesehen,

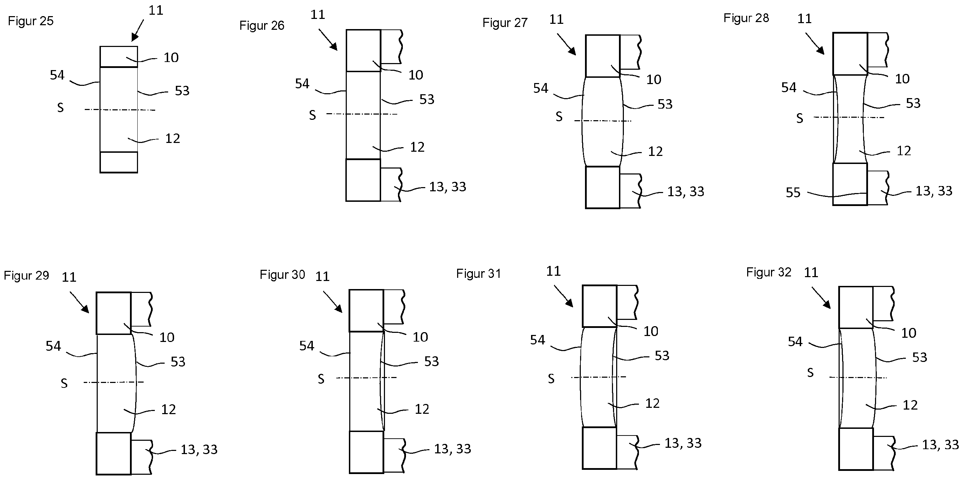

- Figur 25: eine entlang der Symmetrieachse S verlaufende schematische Schnittdarstellung eines hier beschriebenen Fensters zur Erläuterung von dessen Herstellungsverfahren,
- Figur 26: eine entlang der Symmetrieachse S einer Sensoraufnahme zum Halten von zumindest einem Sensor verlaufende schematische Schnittdarstellung mit einem Teil des Halterungskörpers der Sensoraufnahme und einem planparallelen transparenten Element, wobei die Symmetrieachse S lediglich beispielhaft für ein Fenster in Figur 9 dargestellt ist,
- Figur 27: eine entlang der Symmetrieachse S eines Fensters der Sensoraufnahme verlaufende schematische Schnittdarstellung mit einem Teil des Halterungskörpers der Sensoraufnahme und einem bikonvexen transparenten Element,
- Figur 28: eine entlang der Symmetrieachse S eines Fensters der Sensoraufnahme verlaufende schematische Schnittdarstellung mit einem Teil des Halterungskörpers der Sensoraufnahme und einem bikonkaven transparenten Element,
- Figur 29: eine entlang der Symmetrieachse S eines Fensters der Sensoraufnahme verlaufende schematische Schnittdarstellung mit einem Teil des Halterungskörpers der Sensoraufnahme und einem plankonvexen transparenten Element,
- Figur 30: eine entlang der Symmetrieachse S eines Fensters der Sensoraufnahme verlaufende schematische Schnittdarstellung mit einem Teil des Halterungskörpers der Sensoraufnahme und einem plankonkaven transparenten Element,
- Figur 31: eine entlang der Symmetrieachse S eines Fensters der Sensoraufnahme verlaufende schematische Schnittdarstellung mit einem Teil des Halterungskörpers der Sensoraufnahme und einem konvexkonkaven transparenten Element,
- Figur 32: eine entlang der Symmetrieachse S eines Fensters der Sensoraufnahme verlaufende schematische Schnittdarstellung mit einem Teil des Halterungskörpers der Sensoraufnahme und einem konkavkonvexen transparenten Element,

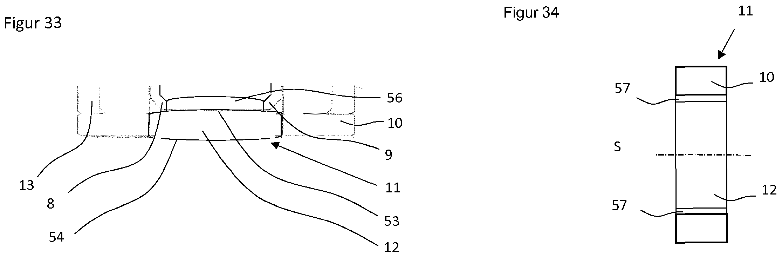

- Figur 33: einen Ausschnitt analog zu der Darstellung der Figur 9, jedoch eine weitere Ausführungsform darstellend, bei welcher ein Fenster einen Teil einer diesem zugeordneten mikroskopischen Einrichtung, insbesondere einer Mikroskopsonde bildet
- Figur 34: eine entlang der Symmetrieachse S verlaufende schematische Schnittdarstellung eines hier beschriebenen Fensters, bei welchem dessen transparentes Element Quarzglas umfasst, zur Erläuterung von dessen Herstellungsverfahren,

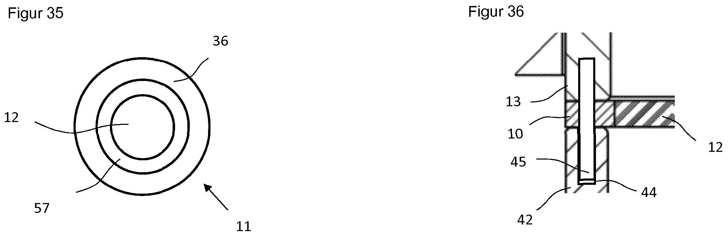

- Figur 35: eine Aufsicht auf das in Figur 23 dargestellte Fester, bei welchem dessen transparentes Element Quarzglas umfasst
- Figur 36: einen Ausschnitt aus Figur 23, welcher dem in Figur 23 dargestellten Feld innerhalb des Rechtecks K entspricht.

### Detaillierte Beschreibung bevorzugter Ausführungsformen

Bei der nachfolgenden detaillierten Beschreibung bevorzugter Ausführungsformen sind gleiche oder gleich wirkende Bestandteile in den Figuren jeweils mit denselben Bezugszeichen versehen.

Die Figuren sind jedoch um der Klarheit willen nicht maßstabsgerecht dargestellt.

Um der Kürze Willen wird nachfolgend der Behälter zur Aufnahme von fluiden Medien, die biologisches Material enthalten, auch als Behälter zur Aufnahme von fluiden Medien oder noch stärker abgekürzt auch nur als Behälter bezeichnet.

Soweit im Rahmen der vorliegenden Offenbarung der Begriff der fluiden Medien verwendet wird, trägt dieses der Tatsache Rechnung, dass in einem Bioreaktor mehr als ein fluider Bestandteil vorhanden sein kann, beispielsweise ein Trägerfluid, in welchem sich das biologische Material oder eine Vorstufe des biologischen Materials befinden kann, in welchem aber auch fluide Bestandteile des biologischen Materials selbst oder weitere Nährlösung vorhanden sein können. Soweit sich jedoch nur ein Trägerfluid und kein weiterer fluider Bestandteil im Bioreaktor befindet, umfasst der Begriff der fluiden Medien auch dieses Trägerfluid im Singular, ohne dass weitere fluide Bestandteile zusätzlich vorhanden sein müssen.

Das biologische Material umfasst im Rahmen der vorliegenden Offenbarung generell pro und eukaryotische Zellen, wie Mammaliazellen, photo-, hetero- und mixotrophe Mikroorganismen und liegt beispielsweise in Form von Mikroalgen, beispielsweise Blaualgen oder Cyanobakterien vor und umfasst insbesondere auch durch Mutagenese veränderten photo- oder mixotrophe Mikroorganismen.

Nachfolgend wird zunächst auf Figur 12 Bezug genommen, welche einem im Ganzen mit dem Bezugszeichen 1 versehenen Multi-Use-Bioreaktor darstellt, an welchem eine ebenfalls im Ganzen mit dem Bezugszeichen 2 bezeichnete Sensoraufnahme zum Halten von zumindest einem Sensor angeordnet ist.

Der Bioreaktor 1 umfasst einen Behälter 3 zur Aufnahme fluider Medien, welche biologisches Material enthalten, wobei sowohl das fluide Medium 4 oder die fluiden Medien 4 als auch das punktförmig dargestellte biologische Material 5 innerhalb des Kreises K, welcher in dessen Inneren eine aufgebrochene Querschnittsdarstellung des Behälter 3 zeigt, zu erkennen sind.

Generell kann der Behälter 3 der Behälter eines Multi-Use-Bioreaktors 1 zur Mehrfachanwendung sein, wie dieser beispielhaft in Figur 12 dargestellt ist, oder auch eines Single-Use-Bioreaktors für Einmalanwendungen.

Vorteilhaft umfasst der Behälter 3 bei einem Multi-Use-Bioreaktor Edelstahl oder besteht dieser Behälter 3 aus Edelstahl. Auch die nachfolgend noch detaillierter beschriebene Vorrichtung 8 kann Edelstahl umfassen oder es können zumindest eines oder mehrere von deren Bestandteilen sowie beispielsweise der in Figur 7 zu erkennende der Grundkörper 10 der hier offenbarten Fenster 11 aus Edelstahl bestehen oder dieses umfassen.

Verwendbar sind hierbei alle Edelstähle, insbesondere auch austenitische und ferritische Edelstähle, bevorzugt jedoch nur soweit diese bei der Durchführung der Erfindung rostfrei bleiben.

Der Edelstahl kann bevorzugt auch 316L Pharmastahl umfassen, oder vollständig aus diesem bestehen.

Ferner sind prinzipiell auch Titan, Tantal, Nickel sowie Monell-Legierung mit hohem Kupferanteil verwendbar, wobei Titan bei Verwendung für die Beleuchtungsvorrichtung insbesondere für deren Gehäusekörper auch emailliert sein kann.

Bei Single-Use-Reaktoren 1 kann der Behälter 3 zur Aufnahme von fluiden Medien, die biologisches Material enthalten, einen Kunststoff, insbesondere einen sterilisierbaren Kunststoff umfassen oder aus einem Kunststoff, insbesondere einem sterilisierbaren Kunststoff bestehen. In diesem Fall hat dann der Behälter 3 nicht die in Figur 12 dargestellte Form sondern kann auch als Beutel ausgebildet sein.

Ferner können an dem Behälter 3 mehr als ein Port 6, welche jeweils auch als Durchführung bezeichnet werden, angeordnet sein.

Bevorzugt umfassen diese Durchführungen 6, insbesondere auch bei der in Figur 12 dargestellten Ausführungsform einen Standardport, wie beispielsweise einen Ingold-Port, einen Broadly-James- Port, einen B.-Braun-Sicherheitsport oder einen einem anderen Standard entsprechenden Port.

Bei Einwegreaktoren oder Single-Use-Reaktoren, wie diese beispielsweise in den Figuren 5 bis 8, 10 und 19 bis 24 dargestellt sind, kann die Durchführung 6 auch einen Tri-Clamp-, einen Sanitary-Clamp-Port oder einen herstellerspezifisch ausgebildeten Port umfassen.

Die Durchführungen 6 weisen jeweils eine Durchgangsöffnung 7 definierten Durchmessers auf, welche in typischer Weise das Innere eines Bioreaktors 1 mit dessen Äußerem verbindet oder den Zugang zum Behälter 3 eröffnet.

Besonders gut sind diese Durchgangsöffnungen 7 der Ports 6 beispielsweise auch jeweils in den Figuren 1, 5, 9, 10 und 11 zu erkennen.

Innerhalb zumindest einer Durchgangsöffnung 7 ist eine Sensoraufnahme 2 zum Halten zumindest eines Sensors 8, beispielsweise einer bilderfassenden Einrichtung oder Mikroskopsonde 9 angeordnet, wobei die Sensoraufnahme 2 sich zumindest teilweise in der Durchgangsöffnung 7 des Ports 6 oder Durchführung 6 erstreckend gehalten ist, denn diese kann bereichsweise sowohl in das Innere des Behälters 3 als auch über die Durchführung 7 hinaus zum Äußeren des Behälters 3 hinausragen.

Die erfindungsgemäße Ausführungsform umfasst somit einen Bioreaktor 1 mit einem Behälter 3 zur Aufnahme von fluiden Medien 4, die biologisches Material 5 enthalten, eine Durchführung 6 oder einen Port 6 mit einer Durchgangsöffnung 7 zwischen dem Inneren des Behälters 3 zur Aufnahme von fluiden Medien 4, die biologisches Material 5 enthalten, und dem Äußeren des Behälters 3 zur Aufnahme von fluiden Medien 4, die biologisches Material 5 enthalten, wobei sich eine Sensoraufnahme 2 zum Halten von zumindest einem Sensor 8 zumindest teilweise in der Durchgangsöffnung 7 der Durchführung 6 erstreckt, wobei die Sensoraufnahme mit deren Fenster die Durchgangsöffnung verschließt und die Sensoraufnahme einen Halterungskörper umfasst, welcher einen Standardflansch aufweist.

Die Sensoraufnahme 2 umfasst einen Halterungskörper 13 mit einem Sensoraufnahmebereich 14, welcher als Durchgangsöffnung ausgebildet ist, an welchem ein Fenster 11 mit einem transparenten Element 12 angeordnet ist, das für elektromagnetische Strahlung transmittierend ist, siehe beispielsweise hierzu auch die Figuren 1 und 5.

Das Fenster 11 umfasst einen Grundkörper 10, welcher Stahl umfasst oder aus Stahl besteht und das transparente Element 12 hält, wie nachfolgend unter Bezugnahme auf die Figuren 25 bis 32 noch detaillierter beschrieben werden wird.

Der Bioreaktor 1 kann auch über mehrere Durchführungen 6 verfügen, in welchen jeweils eine Sensoraufnahme 2 angeordnet ist.

Dabei können jeweils die gleiche Art von Sensor 8 oder können auch verschiedene Ausführungsformen von Sensoren 8 in der Sensoraufnahme 2 angeordnet sein.

Eine erste bevorzugte Ausführungsform umfasst als Sensor 8 beispielsweise eine dem Fachmann an sich bekannte Mikroskopsonde 8, 9 welche über eine bilderfassende Einrichtung verfügen kann, eine im wesentlichen zylindrische Gestalt aufweist und lösbar aber dennoch fest an der Sensoraufnahme 2 gehalten ist.

Nachfolgend wird unter Bezugnahme auf die Figuren 1 bis 4 die Sensoraufnahme 2 zum Halten von zumindest einem Sensor 8 detaillierter beschrieben.

Wie besonders gut aus Figur 1 und Figur 5 zu erkennen ist, umfasst die Sensoraufnahme 2 einen Halterungskörper 13, welcher zylindersymmetrisch, insbesondere säulenförmig und vorzugsweise einstückig ausgebildet ist.

Der Halterungskörper 13 weist eine vorzugsweise zylindrische Durchgangsöffnung 14 auf, welche auf der dem Inneren des Bioreaktors 1 zugeordneten Seite mittels einem Fensters 11 fluiddicht, insbesondere hermetisch dicht abgeschlossen ist.

In den Figuren 13 bis 18 ist eine weitere Ausführungsform eines Multi-Use-Bioreaktors 1 zur Mehrfachverwendung mit einer Sensoraufnahme 2 zum Halten von zumindest einem Sensor und in den Figuren 19 bis 24 eine nochmals weitere Ausführungsform eines Single-Use-Bioreaktors 1 zur Einmalverwendung mit einer Sensoraufnahme 2 dargestellt.

Beide Ausführungsformen umfassen jeweils eine Messkammer 40, insbesondere für Trübungsmessungen, siehe hierzu beispielsweise die Figuren 19 und 20.

Die Messkammer 40 wird durch ein im Querschnitt im Wesentlichen L-förmiges Fußteil 41 zusammen mit dem Fenster 11, insbesondere mit dessen Grundkörper 10 und transparentem Element 12 ausgebildet.

Das Fußteil 41 umfasst einen Sockelabschnitt 42, der vorzugsweise lösbar befestigbar am Halterungsköper 13 gehalten ist und sich in Richtung der Symmetrieachse S erstreckt. Am distalen, somit zum Halterungskörper 13 am meisten beabstandeten Ende des Sockelabschnitts 42 bildet das Fußteil 41 eine plane, sich senkrecht zur Symmetrieachse S erstreckende Endfläche 43 aus, welche die Messkammer 40 in Richtung der Symmetrieachse berandet.

Die Endfläche 43 und das transparente Element 12 sind in einem Abstand T zueinander angeordnet, sodass bei sich bei Verwendung parallelen Lichts, welches sich in Richtung der Symmetrieachse S ausbreitet, aus der Oberfläche des transparenten Elements 12 und dem Abstand T ein Volumen definiert, welches, innerhalb des Behälter 2 zur Aufnahme fluider Medien angeordnet ist und für Trübungsmessungen nutzbar ist.

Falls jedoch kein paralleles, sondern divergentes Licht zur Messung verwendet wird, ist dennoch auch durch die Größe der Oberfläche 43 sowie den Abstand T ein Messvolumen definiert, welches auch bei dieser Ausführungsform für Trübungsmessungen nutzbar ist.

Generell kann jedoch aufgrund der statischen Geometrie stets ein sich nicht änderndes Messvolumen zunächst kalibriert werden, beispielsweise nur mit einer Nährlösung ohne das biologische Material und danach für entsprechende Trübungsmessungen verwendet werden, nachdem das biologische Material 5 in den Bioreaktor 1 eingebracht wurde.

Das Fußteil 42 kann beispielsweise mittels einer Schweißverbindung am Grundkörper 10 des Fensters 11 dauerhaft gehalten sein, siehe beispielsweise Figur 23.

Alternativ kann das Fußteil 41, wie beispielhaft in Figur 36 dargestellt, auch lösbar befestigbar ausgebildet sein. Hierzu kann das Fußteil 41 in dessen Sockelabschnitt 42 eine Bohrung 44 mit definierter Passung aufweisen, in welche sich ein Passstift 45 erstreckt, der fest im Halterungskörper 13 und vorzugsweise auch fest im Grundkörper 10 des Fensters 11 gehalten ist.
Wie beispielsweise auch in Figur 7 gut zu erkennen ist, umfasst das Fenster 11 ein transparentes Element 12, welches in einem Grundkörper 10 fluiddicht, insbesondere hermetisch dicht gehalten ist.

Als hermetisch dicht oder auch fluiddicht wird im Sinne der vorliegenden Offenbarung wird ein Gegenstand oder auch eine Verbindung zwischen zwei Gegenständen, beispielsweise zwischen dem transparentem Element 12 und Grundkörper 10 des Fensters 11, dann angesehen werden, wenn diese bei Raumtemperatur eine Leckrate von weniger als 1x10-3 mbar * l/sec aufweisen, wenn diese einseitig mit He und 1 bar Druckdifferenz beaufschlagt werden.

Das transparente Element 12 ist für elektromagnetische Strahlung transparent, denn dieses umfasst Glas oder besteht aus Glas, wobei das Glas bevorzugt Quarzglas oder Borosilikatglas enthält oder aus diesem besteht.

Der Halterungskörper 13 bildet zusammen mit dem Fenster 11 eine Halterung 15 für den Sensor 8, in welcher der Sensor 8 lösbar befestigbar gehalten ist.

Hierzu kann der Halterungskörper 13, wie beispielsweise auch Figur 1 und Figur 11 zu entnehmen ist, vorzugsweise mittels eines Reibelements 16, vorzugsweise einem O-Ring eine reibschlüssige Verbindung zu dem Sensor 8 eingehen.

Das Reibelement 16 ist in einer zylindrischen Ausnehmung 17 des Halterungskörpers 13 durch ein im Wesentlichen ringförmiges Druckelement 18 gehalten, welches in axialer Richtung des Halterungskörpers 13 eine definiert einstellbare Kraft auf das Reibelement ausübt.

Durch die dabei entstehende definierte Deformation des Reibelements 16 kann der Sensor 8 mit einer daraus resultierenden definierten Kraft innerhalb der Durchgansöffnung 14 gehalten werden, welche betriebssicher dessen Lage sicherstellt aber dennoch eine zügige manuelle Entnahme aus der Durchgangsöffnung 14 oder auch ein zügiges manuelles Einbringen in die Durchgangsöffnung 14 ermöglicht.

Mittels eines Sprengrings 19 ist das ringförmige Druckelement 18 in seiner Lage definiert fixiert.
Der Halterungskörper 13 selbst liegt mit einem seitlichen, sich in radialer Richtung erstreckenden Absatz 20 an einem oberen Flansch 21 der Durchführung 6 formschlüssig an.

Die Sensoraufnahme 2 erstreckt sich somit innerhalb der Durchführung 6 oder der Durchgangsöffnung 7 des Ports 6 formschlüssig zur Durchführung oder formschlüssig zur Durchgangsöffnung 7 des Ports 6.

Mittels einer Hutmutter 22 mit einer zylindrischen Durchgangsöffnung 23, welche den sich in radialer Richtung erstreckenden Absatz 20 übergreift, ist der Halterungskörper 13 lösbar, jedoch ortsfest an dem Port 6 gehalten.

Hierzu weist sowohl die Hutmutter 22 ein Gewinde 24 als auch der Halterungskörper 13 ein Gegengewinde 25 auf.

Mittels eines Sprengrings 26 ist die Hutmutter 22 drehbar jedoch axial nur mit geringem Spiel unverlierbar am Halterungskörper 13 gehalten.

Durch Verdrehen der Hutmutter 22 kann die Sensoraufnahme 2 zügig an dem jeweiligen Port 6 betriebssicher montiert oder auch von diesem gelöst werden.

Mittels eines Dichtelements 27, beispielsweise eines O-Rings, ist der Halterungskörper 13 fluiddicht und vorzugsweise hermetisch dichtend gegenüber der Durchgangsöffnung 7 des Behälters 3 form- und reibschlüssig in der Durchgangsöffnung 7 gehalten.

Hierbei definiert die Symmetrieachse S des Halterungskörpers 13, welche beispielsweise auch in den Figuren 1 und 5 zu erkennen ist, die axiale oder Längsrichtung, auf welche jeweils im Rahmen der vorliegenden Offenbarung Bezug genommen wird.

Bei einer in die Durchgangsöffnung 7 eingesetzten und vorzugsweise wie vorstehend beschrieben fixierten Sensoraufnahme 2, siehe beispielsweise Figur 9, wird der axiale Abstand 29 des Fensters 11 und somit des transparenten Elements 12 zur Innenwand 28 des Bioreaktors 1 durch den axialen Abstand 30 des Fensters 11 zum seitlichen Absatz 20 definiert.

Der axiale Abstand des Fensters 11 zum seitlichen Absatz 20 wird dabei von der Unterseite des seitlichen, sich in radialer Richtung erstreckender Absatzes 20 ausgehend, welches durch eine Hilfslinie H angedeutet ist, bis zur Oberseite des Grundkörpers 10 des Fensters 11 gemessen, wie dieses beispielsweise in Figur 9 zu erkennen ist.

Der axiale Abstand 29 des Fensters 11 zur Innenwand 28 des Bioreaktors 1 ist hierbei der größte Abstand zwischen der Unterseite des Grundkörpers 11 zur Innenwand 28 des Bioreaktors 1, somit zur Innenwand 28 des Behälters 3 des Bioreaktors 1.

Bevorzugt ist hierbei das transparente Element 12 des Fensters 11 bei in die Durchgangsöffnung 7 eingesetztem Halterungskörper 13, insbesondere bei Anlage des seitlichen Absatzes 20 an einem Standardport 6, wie beispielsweise in Figuren 1 und 9 gezeigt, innerhalb des Bioreaktors 1 angeordnet.

Ein Satz von Halterungskörpern 13 mit verschiedenem axialem Abstand 30 des transparenten Elements 12 des Fensters 11 zum seitlichen Absatz 20 kann hierbei eine wählbare axiale Positionierung des transparenten Elements 12 des Fensters innerhalb des Bioreaktors 1 ermöglichen.

Hierdurch können bei Verwendung mehrerer Vorrichtungen 2 mit jeweils verschiedenem axialen Abstand 30 des Fensters 11 zum seitlichen Absatz 20 jeweils verschiedene Orte des Bioreaktors 1 erfasst werden. Somit kann durch dieses Vorgehen ein Bioreaktor 1 bei Verwendung mehrerer Sensoraufnahmen 2 in dessen lokalen Abläufen jeweils erheblich besser erfasst werden.

Nachfolgend wird auf Figur 5 und 10 Bezug genommen, welche eine zweite bevorzugte Ausführungsform einer Sensoraufnahme 2 zeigen, welche an einem Single-Use-Bioreaktor 1 angeordnet ist, bei welchem der Behälter 3 des Bioreaktors aus Kunststoff besteht.

Bei dieser Ausführungsform ist die Sensoraufnahme 2, insbesondere mit deren Halterungskörper 13 mit dem Behälter des Bioreaktors 1 verschweißt, insbesondere ohne Zusatzstoffe mit der Durchgangsöffnung 7 des Bioreaktors 1 verschweißt.

Um die Oberfläche zu erhöhen und die Haftkraft zu verbessern, kann die Außenseite des Halterungskörpers 13, an welcher der Kunststoff des Bioreaktors 1 anliegt eine Randrierung aufweisen.

Wie besonders gut der Figur 10 zu entnehmen ist, weist der Halterungskörper 13 einen Standardflansch auf, welcher sich radial nach außen erstreckt und in axialer Richtung eine Anlagefläche 32 für ein Dichtmittel 33 definiert.

Ferner umfasst bei dieser Ausführungsform die Sensoraufnahme 2 eine Abdeckkappe 34 mit einer Anlagefläche 35 für ein Dichtmittel 33 zugeordnet ist, welche Teil eines Sensors 8 sein kann und vorzugsweise einen Sensor 8 in oder an der Sensoraufnahme 2 halten kann.

Diese Abdeckkappe 34 kann, wie es einem Fachmann bekannt, mit üblichen Verschluß- und Haltemitteln für Tri-Clamp-Ports am Flansch 31 unter Zwischenlage des Dichtmittels 33 gehalten sein. Hierzu verwendbare Verschluss-Klammern sind beispielsweise genormt nach ISO 2852 oder DIN 32676. Dieser in den Figuren 9 und 10 lediglich beispielhaft dargestellte Sensor 8 kann beispielsweise einen ersten Sensorabschnitt 36 umfassen, welcher in die Durchgangsöffnung 14 des Halterungskörpers 13 vorzugsweise formschlüssig einbringbar ist und zumindest eine sensorische Einrichtung umfasst.

Die sensorische Einrichtung kann beispielsweise eine Mikroskopsonde 9 sein, welche ein bildgebendes optisches System umfasst, von welchem in Figur 33 beispielhaft das optische Linsenelement 56 dargestellt ist.

In weiterer Ausgestaltung kann die Mikroskopsonde 9 eine bildaufnehmende Einrichtung umfassen, beispielsweise in Form eines bildaufzeichnenden Photosensors.

Alternativ oder zusätzlich kann die sensorische Einrichtung einen Bildleiter, insbesondere einen Faser-Bildleiter 37 umfasst, welcher in Figur 9 lediglich beispielhaft und stark schematisiert dargestellt ist.

Alternativ kann die sensorische Einrichtung ein Spektrometer 38 umfassen, welches beispielhaft als Spektrometerkopf 39 dargestellt ist, der an den Bildleiter 37 angeflanscht und mit diesem optisch verbunden ist.

Wenn jedoch die Sensoraufnahme 2 zumindest einen Sensor 8, insbesondere wie vorstehend erwähnt, innerhalb einer Mikroskopsonde 8 oder dieser Mikroskopsonde 8 zugeordnet eine bildaufzeichnende Einrichtung aufweist, und im Betriebszustand die Strahlungsintensität und/oder Wellenlänge der elektromagnetischen Strahlung im Innern des Bioreaktors 1 insbesondere im Inneren des Behälters 3 mit dieser bilderfassenden Einrichtung aufgenommen wird, kann hierbei die Strahlungsintensität und/oder die Wellenlänge ortsaufgelöst gemessen werden. Beispielhaft sind zur Messung der Wellenlänge nachfolgend auch Beschichtungen auf dem transparenten Element 12 beschrieben. Ferner können innerhalb einer Mikroskopsonde 8 angeordnete Filter sowohl optischer als auch elektronischer Art beispielsweise in der bildaufzeichnenden Einrichtung hierzu verwendet werden.

Eine Prozesskontrolle mittels eines in-situ Mikroskops als Sensor 8 ermöglicht hierbei die Erfassung quantitativer und morphologischer Informationen über die jeweiligen Zellen des biologischen Materials 5.

Daraus abgeleitet kann sich auch direkt die Optimierung der Einflussgrößen für eine optimierte oder zumindest verbesserte Ausbeute ergeben. Diese sind beispielsweise auch die Begasungsrate, die Begasungszusammensetzung, Temperatur, pH, rX, Konzentrationen der gelösten Gase, Durchmischung/Rührerdrehzahl, Feedrate, Feedzusammensetzung, Kultivierungszeit, aktivierende oder inhibierende Faktoren und können auch noch weitere Einflussgrößen umfassen.

Bei den vorliegend bevorzugten Ausführungsformen bildet ein Fenster 11 oder bilden beide Fenster 11 jeweils eine Einglasung für ein transparentes Element 12, vorzugsweise eine GTMS-Druckeinglasung, welche auch als GTMS-Druckverglasung bezeichnet wird, aus, wie dieses nachfolgend unter Bezugnahme auf Figur 25 beschrieben wird.

Figur 25 zeigt eine entlang der Symmetrieachse S verlaufende schematische Schnittdarstellung eines hier beschriebenen Fensters 11 zur Erläuterung von dessen Herstellungsverfahren.

Das in Figur 25 dargestellte Fenster 11 umfasst einen aus Stahl bestehenden, ring- oder zylinderförmigen Grundkörper 10, welcher das transparente Element 12 seitlich umgibt und auf dieses eine Druckkraft ausübt, die eine dauerhaft hermetische und für die Zwecke der vorliegenden Erfindung ausreichend druck- sowie temperaturfeste Verbindung zwischen dem transparenten Element 12 und dem Grundkörper 10 sicherstellt.

Das transparente Element des Fensters ist mittels der GTMS-Druckverglasung an dem Grundkörper 10 und dieser am Halterungskörper 13 der Sensoraufnahme gehalten.

Vorteilhaft ist hierbei das Fenster 11 mittels -Schweißen, insbesondere Laser-Schweißen hermetisch dicht mit dem Halterungskörper 13 verbunden.

Bei seiner Herstellung wird das aus Glas bestehende oder Glas umfassende transparente Element vorzugsweise in etwa bereits seiner endgültigen Form entsprechend innerhalb des Grundkörpers 10 angeordnet und mit diesem zusammen so lange erwärmt, bis das Glas des transparenten Elements 12 dessen Glastemperatur Tg oder dessen Halbkugeltemperatur überschritten hat und beginnt, am Grundkörper 10 anzuschmelzen.

Nach erfolgtem Anschmelzen wird dann der Verbund aus transparentem Element 12 und Grundkörper 10 auf Raumtemperatur abgekühlt, wodurch jeweils ein Fenster 11 mit einem im Wesentlichen scheibenförmigen transparentem Element 12 ausgebildet wird.

Da der thermische Ausdehnungskoeffizient des Edelstahls des Grundkörpers 10 größer als der des Glases des transparenten Elements 12 ist, übt dieser, sobald das Glas des transparenten Elements sich zu verfestigen beginnt, eine mit weiter abnehmender Temperatur ansteigende Druckspannung auf das Glas des transparenten Elements 12 aus.

Der Grundkörper 10 hält das transparente Element 12 dann, nach erfolgter Abkühlung, mit dieser quasi eingefrorenen Druckspannung dauerbetriebsfest hermetisch dicht und temperaturstabil.

Eine derartige Druckverglasung wird im Rahmen der vorliegenden Offenbarung auch als Glas-Metall-Verbindung oder Glas-to-Metal-Seal, GTMS, oder GTMS-Druckverglasung bezeichnet.

Bei einer derartigen Glass-to-Metall-Verbindung übt das Metall über den gesamten Betriebstemperaturbereich Druckkräfte auf das Glas aus, insbesondere auch bei Temperaturen bis zumindest 121° C, vorzugsweise sogar 141° Celsius, welche eine Druckspannung zwischen dem Metall und dem Glas ausbilden und dazu beitragen, dass die Glass-Metall-Verbindung dauerhaft betriebssicher sowohl fluidals auch hermetisch dicht ist.

Ferner treten bei derartigen Glas-Metall-Verbindungen in der Regel keine Spalte auf, wie diese bei Verwendung von herkömmlichen Dichtmitteln, beispielsweise von O-Ringen auftreten können und Raum für Verunreinigungen bieten, welche häufig nur schwer entfernbar sind.

Vorteilhaft ist hierbei ein Unterschied der thermischen Ausdehnungskoeffizienten, welcher die Druckspannung zwischen dem Glas des transparenten Elements 35 und dem Metall des ring- oder zylinderförmiger Grundkörper 36 des Fensters 8 zumindest über den Betriebstemperaturbereich zuverlässig aufrechterhält.

Dieser Unterschied des Ausdehnungskoeffizienten CTE_{M} des Metalls und dem Ausdehnungskoeffizienten des Glases CTE_{G} des transparenten Elements 3 kann beispielsweise kleiner als 80 * 10⁻⁶ /K, bevorzugt kleiner als 30 * 10⁻⁶ /K oder besonders bevorzugt auch kleiner als 20 * 10⁻⁶ /K betragen. Hierbei soll der thermische Ausdehnungskoeffizient des Metalls CTE_{M} jeweils größer als der thermische Ausdehnungskoeffizient des Glases CTE_{G} sein. In allen Fällen soll dieser Unterschied vorzugsweise jedoch mindestens 1 mal 10⁻⁶ /K betragen.

Quarzglas weist beispielsweise ein CTE_{G} von 0,6 * 10⁻⁶ /K auf und kann beispielsweise mit Edelstählen mit einem CTE_{M} von 17-18 *10⁻⁶ /K kombiniert werden.

Der Bioreaktor 1 sowie Vorrichtung zur Halterung einer bilderfassenden Einrichtung 2 ist jeweils einzeln eigenständig oder es sind der Photobioreaktor 1 sowie Vorrichtung 2 zur Halterung einer bilderfassenden Einrichtung auch zusammen autoklavierbar. Dies bedeutet, dass insbesondere die Vorrichtung zur Halterung einer bilderfassenden Einrichtung mit deren hier offenbarten Fenstern 11 hermetisch dicht ist und dabei einer Behandlung mit Sattdampf einer Temperatur von 121° C, insbesondere auch von 141° C standhält, ohne dass der Sattdampf oder durch diesen gebildete Fluide in die Vorrichtung 2 eintreten können.

Autoklavierbar wird im Rahmen dieser Offenbarung auch als autoklavierbar im Sinne der DIN EN ISO 14937; EN ISO 17665, welche für Medizinprodukte gültig ist, verstanden.

Hierdurch wird auch das dem Fachmann bekannte und vorteilhafte Steaming-in-Place ermöglicht.

In diesem dauerbetriebsfest hermetisch dicht und temperaturstabil gehaltenen Zustand kann das Glas des transparenten Elements 12, vorzugsweise nach Prüfung der jeweiligen seitlichen Hauptoberfläche 53, 54 entweder direkt verwendet oder weiteren Oberflächen-bearbeitenden Verfahren, wie beispielsweise Polieren oder formgebenden Schleifen zugeführt werden.

Auf diese Weise kann das jeweilige transparente Element 12 planparallele seitliche Hauptoberflächen 53 und 54 ausbilden oder kann das transparente Element 12 auch plankonvex, plankonkav, bikonvex, bikonkav, konvexkonkav oder konkavkonvex geformt werden, wie dieses den Schnittdarstellungen der Figuren 26 bis 32 zu entnehmen ist.

Bei geringeren optischen Anforderungen an die Oberflächenqualität, insbesondere für zur Messung mit der in Bezug auf Figur 10 beschriebenen Ausführungsform verwendete Strahlengänge, kann das transparente Element 12 auch während des Herstellungsverfahrens in einer entsprechenden Negativ-Form gehalten sein, welche im Wesentlichen bereits dessen finaler Form entspricht.

Die Hauptoberflächen 53 und 54 können eine Beschichtung aufweisen, welche wellenlängenselektiv ist und derart ein optisches Bandpass- oder Kantenfilter ausbilden. Diese Beschichtung kann jeweils nur auf einer oder auch auf beiden Hauptoberflächen 53, 54 vorhanden sein.

Ohne eine derartige, insbesondere ohne jegliche Beschichtung weist das transparente Element 12 zumindest eines Fensters 11 in einem Spektralbereich mit einer Wellenlänge von 250 bis 2000 nm eine Transmission auf, die höher ist als 80%, besonders bevorzugt höher als 90 %.

Wenn das transparente Element 12 des Fensters 11, wie es beispielsweise in den Figuren 34 und 35 gezeigt ist, aus Quarzglas besteht oder Quarzglas umfasst, kann anstelle einer Laserschweißnaht auch ein weiteres Glas 57 oder Glaslot 57, insbesondere bleifreies Glas 57 oder Glaslot 57 verwendet werden, um das Quarzglas hermetisch dicht mit dem ring- oder zylinderförmiger Grundkörper 10 des Fensters 11, insbesondere fluid- und hermetisch dicht zu verbinden.

Bevorzugt wird der Grundkörper 10 der Fenster 11 direkt, insbesondere mittels Schweißen, bevorzugt Laser-Schweißen, jeweils hermetisch dicht mit dem Halterungskörper 13 und/oder dem Gehäuse 33 der Beleuchtungsvorrichtung 31 verbunden, sodass hierdurch das Gehäuse 33 hermetisch dicht ausgebildet ist und der Halterungskörper 13 an dessen unterem Ende hermetisch dicht gegenüber dem Inneren des Behälters 3 ebenfalls hermetisch dicht ausgebildet ist.

Beispielhaft ist die hierbei jeweils entstehende Laserschweißnaht lediglich in Figur 28 mit dem Bezugszeichen 55 dargestellt.

Alternativ kann der Halterungskörper 13 auch einen hochtemperaturbeständigen Kunststoff, insbesondere einen thermoplastischen Kunststoff wie einen Polyaryletherketon, insbesondere einen Polyetheretherketon, PEEK, umfassen oder aus diesem bestehen.

Wenn der Halterungskörper 13 einen hochtemperaturbeständigen Kunststoff, insbesondere einen thermoplastischen Kunststoff wie einen Polyaryletherketon, insbesondere einen Polyetheretherketon, PEEK, umfasst oder aus diesem besteht, muss dieser Halterungskörper 13 nicht unbedingt vollständig hermetisch sein, wie dieses im Rahmen der vorliegenden Offenbarung beschrieben wurde, sondern können dennoch durchaus wertvolle Betriebs- und Einsatzzeiten erreicht werden.

Es kann beispielsweise der PEEK umfassende oder aus PEEK bestehende Halterungskörper 13 eine vorzugsweise säulenförmige Durchgangsöffnung mit kreisförmigem Querschnitt aufweisen und der Außendurchmesser eines transparenten Elements 12 mit ebenfalls kreisförmigen seitlichen Außenabmessungen um etwa 1/10 größer sein als der Innendurchmesser der kreisförmigen Durchgangsöffnung des Halterungskörpers 13. Wenn der Halterungskörper 3 auf eine Temperatur von etwa 200°C erhitzt wird, kann dann das transparente Element 12 in diese Durchgangsöffnung eingesetzt werden und es entsteht bei Abkühlung eine wie vorstehend beschriebene Druckspannung, beispielsweise von etwa 38 MPa, welche noch deutlich unter der Streckgrenze von 110 MPa von PEEK liegt.

Eine weitere bevorzugte Ausführungsform ergibt sich, wenn beispielsweise das in Figur 27 dargestellte Fenster zusammen mit einer Mikroskopsonde 9 verwendet wird, wie dieses beispielsweise der Figur 33 zu entnehmen ist, und dabei dieses Fenster 11 einen Teil der diesem zugeordneten mikroskopischen Einrichtung, nämlich der Mikroskopsonde 8 bildet.

Das transparente Element 12 des Fensters 11 ist bei dieser Ausführungsform Teil eines insbesondere diesem zugeordneten bildgebenden Systems.

Hierbei kann beispielsweise bereits die untere Hauptoberfläche 54 oder auch beide Hauptoberflächen 54, 53 strahl- oder wellenformende Wirkung entfalten und lassen sich prinzipiell höhere numerische Aperturen für den bildgebenden Strahlengang erreichen, da dem ersten optische Linsenelement 56 der Mikroskopsonde 8 ein größerer effektiver Winkelbereich für die eintretende elektromagnetische Strahlung zur Verfügung gestellt werden kann.

Hierdurch kann nicht nur die Auflösung der Mikroskopsonde 8 erhöht, sondern auch die insgesamt zur Verfügung gestellte Intensität der elektromagnetischen Strahlung gesteigert werden, wodurch sich bei Verwendung dieses in Figur 27 dargestellten Fensters, insbesondere auch für die in Figur 10 dargestellte Ausführungsform, ein verbesserter Signalzu-Rausch-Abstand für die hierbei erhaltenen und beispielsweise in elektrische Signale gewandelten Messergebnisse ergibt.

Die Erfindung betrifft generell eine Sensoraufnahme für einen Bioreaktor sowie einen Bioreaktor mit einer Sensoraufnahme und Verfahren zur Vermehrung oder Kultivierung biologischen Materials, bei welchen sich eine Sensoraufnahme zum Halten von zumindest einem Sensor mit einem Fenster zumindest teilweise in einer Durchgangsöffnung eines Behälters zur Aufnahme von fluiden Medien, die biologisches Material enthalten, erstreckt, wobei die Sensoraufnahme mit deren Fenster vorzugsweise die Durchgangsöffnung verschließt, Verwendung findet.

### Bezugszeichenliste

- 1: Bioreaktor
- 2: Sensoraufnahme zum Halten von zumindest einem Sensor
- 3: Behälter zur Aufnahme fluider Medien, die biologisches Material enthalten
- 4: das fluide Medium oder die fluiden Medien
- 5: biologisches Material
- 6: Port oder Durchführung
- 7: Durchgangsöffnung des Behälters 3
- 8: Sensor, insbesondere beispielhaft Mikroskopsonde
- 9: bilderfassenden Einrichtung, insbesondere einer mikroskopischen Einrichtung
- 10: Grundkörper des Fensters 11
- 11: Fenster
- 12: Transparentes Element
- 13: Halterungskörper
- 14: Durchgangsöffnung
- 15: Halterung
- 16: Reibelement, vorzugsweise O-Ring
- 17: Zylindrische Ausnehmung
- 18: Ringförmiges Druckelement
- 19: Sprengring
- 20: seitlicher, sich in radialer Richtung erstreckender Absatz
- 21: oberer Flansch
- 22: Hutmutter
- 23: Durchgangsöffnung
- 24: Gewinde
- 25: Gegengewinde
- 26: Sprengring
- 27: Dichtelement, insbesondere O-Ring
- 28: Innenwand des Bioreaktors 1
- 29: axialer Abstand des Fensters 11 zur Innenwand 28
- 30: axialer Abstand des Fensters 11 zum seitlichen Absatz 20
- 31: Flansch
- 32: Anlagefläche für ein Dichtmittel 33
- 33: Dichtmittel
- 34: Abdeckkappe
- 35: Anlagefläche für ein Dichtmittel, insbesondere das Dichtmittel 33
- 36: Erster Sensorabschnitt
- 37: Faser-Bildleiter
- 38: Spektrometer
- 39: Spektrometerkopf
- 40: Messkammer
- 41: L-förmiges Fußteil
- 42: Sockelabschnitt
- 43: Löt- oder Schweißverbindung
- 44: Bohrung mit definierter Passung
- 45: Passstift

- 53: Hauptoberfläche
- 54: Hauptoberfläche
- 55: Laserschweißnaht
- 56: Optisches Linsenelement
- 57: weiteres Glas oder Glaslot, insbesondere bleifreies Glas oder Glaslot
- S: Symmetrieachse
- H: Hilfslinie
- K: Rechteck, einen Ausschnitt aus Figur 23 definierend

## Patentansprüche

1. Bioreaktor umfassend
einen Behälter zur Aufnahme von fluiden Medien, die biologisches Material enthalten,
eine Durchführung mit einer Durchgangsöffnung zwischen dem Inneren des Behälters zur Aufnahme von fluiden Medien, die biologisches Material enthalten, und dem Äußeren des Behälters zur Aufnahme von fluiden Medien, die biologisches Material enthalten, **dadurch gekennzeichnet, dass**
sich eine Sensoraufnahme zum Halten von zumindest einem Sensor zumindest teilweise in der Durchgangsöffnung der Durchführung erstreckt, wobei die Sensoraufnahme mit deren Fenster die Durchgangsöffnung verschließt
und die Sensoraufnahme einen Halterungskörper umfasst, welcher einen Standardflansch aufweist.

2. Bioreaktor nach Anspruch 1, **dadurch gekennzeichnet, dass** der Bioreaktor zusammen mit der Sensoraufnahme zum Halten von zumindest einem Sensor autoklavierbar ist, insbesondere während diese zumindest teilweise in der Durchgangsöffnung der Durchführung gehalten ist, autoklavierbar ist.

3. Sensoraufnahme zum Halten von zumindest einem Sensor für einen Bioreaktor, insbesondere für einen Bioreaktor mit den Merkmalen einer der vorstehenden beiden Ansprüche, **gekennzeichnet durch** einen Halterungskörper mit einem Sensoraufnahmebereich, an welchem ein Fenster mit einem transparenten Element angeordnet ist, das für elektromagnetische Strahlung transmittierend ist,
wobei der Halterungskörper einen Standardflansch umfasst.

4. Sensoraufnahme für einen Bioreaktor nach Anspruch 3, **dadurch gekennzeichnet, dass** der Halterungskörper zylindersymmetrisch, insbesondere säulenförmig ausgebildet ist und eine Durchgangsöffnung aufweist, welche auf der dem Inneren des Bioreaktors zugeordneten Seite mittels des Fensters fluiddicht, insbesondere hermetisch dicht abgeschlossen ist, wobei vorzugsweise das transparente Element des Fensters in einem Spektralbereich mit einer Wellenlänge von 250 bis 2000 nm eine Transmission aufweist, die höher ist als 80%, besonders bevorzugt höher als 90 %.

5. Sensoraufnahme für einen Bioreaktor nach Anspruch 3 oder 4, **dadurch gekennzeichnet, dass** das transparente Element des Fensters Glas umfasst oder aus Glas besteht und
vorzugsweise Quarzglas oder Borosilikatglas umfasst oder aus diesem besteht.

6. Sensoraufnahme für einen Bioreaktor nach einem der Ansprüche von 3 bis 5, **dadurch gekennzeichnet, dass** das transparente Element des Fensters mittels einer GTMS-Druckverglasung an einem Grundkörper und dieser am Halterungskörper der Sensoraufnahme gehalten ist, und/oder das Fenster mittels -Schweißen, insbesondere Laser-Schweißen hermetisch dicht mit dem Halterungskörper verbunden ist.

7. Sensoraufnahme für einen Bioreaktor nach einem der Ansprüche von 3 bis 6, **dadurch gekennzeichnet, dass** das transparente Element des Fensters scheibenförmig ausgebildet ist und insbesondere planparallele Hauptoberflächen aufweist oder plankonvex, plankonkav, bikonvex, bikonkav, konvexkonkav oder konkavkonvex geformt ist.

8. Sensoraufnahme für einen Bioreaktor nach einem der Ansprüche von 3 bis 7, **dadurch gekennzeichnet, dass** das transparente Element des Fensters Teil eines diesem zugeordneten bildgebenden Systems ist.

9. Sensoraufnahme für einen Bioreaktor nach einem der Ansprüche von 3 bis 8, **dadurch gekennzeichnet, dass** der Halterungskörper einen seitlichen, sich in radialer Richtung erstreckenden Absatz aufweist und vorzugsweise bei in die Durchgangsöffnung eingesetzter Sensoraufnahme der axiale Abstand des transparenten Elements des Fensters zur Innenwand des Bioreaktors durch den axialen Abstand des transparenten Elements des Fensters zum seitlichen Absatz definiert ist.

10. Sensoraufnahme für einen Bioreaktor nach einem der Ansprüche von 3 bis 9, **dadurch gekennzeichnet, dass** der Standardflansch sich radial nach außen erstreckt und in axialer Richtung eine Anlagefläche für ein Dichtmittel definiert.

11. Sensor für eine Sensoraufnahme nach einem der Ansprüche von 3 bis 10, **dadurch gekennzeichnet, dass**, der Sensor zumindest einen ersten Sensorabschnitt, welcher in die Durchgangsöffnung des Halterungskörpers formschlüssig einbringbar ist, und zumindest eine sensorische Einrichtung umfasst.

12. Sensor für eine Sensoraufnahme nach Anspruch 11, **dadurch gekennzeichnet, dass** die sensorische Einrichtung einen Bildleiter, insbesondere einen Faser-Bildleiter umfasst und/oder die sensorische Einrichtung ein bildgebendes optisches System, insbesondere zusammen mit einer bildaufnehmenden Einrichtung umfasst und/oder die sensorische Einrichtung ein Spektrometer umfasst.

13. Verfahren zur Vermehrung oder Kultivierung biologischen Materials umfassend das Einbringen fluiden, insbesondere biologischen Materials oder einer Vorstufe biologischen Materials in einen Bioreaktor nach Anspruch 1 oder 2,
das Erfassen einer physikalischen, chemischen oder biologischen Messgröße unter Verwendung einer Sensoraufnahme mit den Merkmalen der Ansprüche von 3 bis 10 sowie eines Sensors mit den Merkmalen des Anspruchs 11 oder 12,
umfassend insbesondere die Herstellung von Pharmazeutika, insbesondere von Biopharmazeutika.

14. Verfahren zur Vermehrung oder Kultivierung nach Anspruch 13, bei welchem der Bioreaktor nach dem Anbringen der Sensoraufnahme sterilisiert wird, und/oder
der Bioreaktor nach Anbringen der Sensoraufnahme, mit einem Sensor bestückt wird, wobei der Sensor zumindest abschnittsweise in den Sensoraufnahmebereich des Halterungskörpers eingebracht wird.

15. Verfahren nach Anspruch 13 oder 14, bei welchem die Sensoraufnahme zumindest einen Sensor aufnimmt, und im Betriebszustand die Strahlungsintensität und/oder die Wellenlänge der elektromagnetischen Strahlung im Innern des Bioreaktors, insbesondere im Innern des Behälters zur Aufnahme fluider Medien, welche biologisches Material enthalten, gemessen, insbesondere ortsaufgelöst gemessen wird und insbesondere
für einen definierten Zeitraum elektromagnetische Strahlung einer definierten Wellenlänge, bevorzugt 250 nm in den Bioreaktor eingestrahlt wird und nach dieser Einstrahlung breitbandig oder selektiv bei einer Wellenlänge, insbesondere bei 270 nm eine Strahlungsintensität und/oder Wellenlänge der elektromagnetischen Strahlung im Innern des Bioreaktors gemessen wird.

16. Verfahren nach einem der Ansprüche von 13 bis 15, bei welchem während der Kultivierung biologischen Materials im Behälter (3) des Bioreaktors (1) ein Sensor (8) gewechselt wird,
wobei die Sterilitätsbedingungen innerhalb des Behälters (3) des Bioreaktors (1) während eines Austauschs oder Wechsel des Sensors (8) aufrecht erhalten werden.

17. Verfahren nach einem der Ansprüche von 13 bis 16, bei welchem durch Mutagenese veränderte photo- oder mixotrophen Mikroorganismen, insbesondere auch Mikroalgen, Hefen und Bakterien verwendet werden.

## Claims

1. A bioreactor, comprising
a container for holding fluid media containing biological material;
a feedthrough with a through-opening between the interior of the container for holding fluid media containing biological material and the exterior of the container for holding fluid media containing biological material;
**characterised in that**
a sensor receptacle for supporting at least one sensor extends at least partially in the through-opening of the feedthrough, wherein the sensor receptacle has a window that seals the through-opening; and wherein the sensor receptacle has a holder body which has a standard flange.

2. The bioreactor according to claim 1, **characterised in that** the bioreactor is autoclavable together with the sensor receptacle for supporting at least one sensor, in particular autoclavable while the sensor receptacle is held at least partially in the through-opening of the feedthrough.

3. A sensor receptacle for supporting at least one sensor for a bioreactor, in particular for a bioreactor with the features of any one of the two preceding claims, **characterised by** a holder body with a sensor receiving area, on which a window is arranged which includes a transparent element that is transmissive to electromagnetic radiation, wherein the holder body comprises a standard flange,

4. The sensor receptacle for a bioreactor according to claim 3, **characterised in that** the holder body has a cylindrically symmetrical shape, in particular a columnar shape, and has a through-opening which is sealed fluid-tightly, in particular hermetically by the window on the side associated with the interior of the bioreactor;
wherein, preferably, the transparent element of the window exhibits a transmittance of greater than 80 %, most preferably greater than 90 %, in a spectral range of wavelengths between 250 and 2000 nm.

5. The sensor receptacle for a bioreactor according to claim 3 or 4, **characterised in that** the transparent element of the window comprises glass or is made of glass, and preferably comprises or is made of fused silica glass or borosilicate glass.

6. The sensor receptacle for a bioreactor according to any one of claims 3 to 5, **characterised in that** the transparent element of the window is secured to a main body by a glass-to-metal seal (GTMS) compression glass seal and the main body is secured to the holder body of the sensor receptacle; and/or that the window is connected to the holder body in a hermetically sealing manner by welding, in particular laser welding.

7. The sensor receptacle for a bioreactor according to any one of claims 3 to 6, **characterised in that** the transparent element of the window has a sheet-like shape and in particular has plane-parallel main surfaces or a plano-convex, plano-concave, biconvex, biconcave, convexo-concave, or concavo-convex shape.

8. The sensor receptacle for a bioreactor according to any one of claims 3 to 7, **characterised in that** the transparent element of the window forms part of an imaging system associated therewith.

9. The sensor receptacle for a bioreactor according to any one of claims 3 to 8, **characterised in that** the holder body has a radially extending lateral shoulder, and wherein, preferably, when the sensor receptacle is installed in the through-opening, the axial distance between the transparent element of the window and the inner surface of the bioreactor is defined by the axial distance of the transparent element of the window to the lateral shoulder.

10. The sensor receptacle for a bioreactor according to any one of claims 3 to 9, **characterised in that** the standard flange extends radially outward and defines a contact surface for a sealing means in the axial direction.

11. A sensor for a sensor receptacle according to any one of claims 3 to 10, **characterised in that** the sensor has at least a first sensor portion which can be fitted into the through-opening of the holder body in a positively fitting manner, and which comprises at least one sensory device.

12. The sensor for a sensor receptacle according to claim 11, **characterised in**
**that** the sensory device comprises an image guide, in particular a fibre image guide; and/or that the sensory device comprises an imaging optical system, in particular in combination with an image capturing device; and/or
**that** the sensory device comprises a spectrometer.

13. A method for propagation or cultivation of biological material, comprising
the introducing of fluid, in particular biological material or of a precursor of biological material into a bioreactor according to claim 1 or 2;
the capturing of a physical, chemical, or biological parameter using a sensor receptacle with the features of claims 3 to 10 and a sensor with the features of claim 11 or 12,
comprising in particular the preparation of pharmaceuticals, in particular biopharmaceuticals.

14. The method for propagation or cultivation of biological material according to claim 13, wherein the bioreactor is sterilized after the sensor receptacle has been mounted thereto; and/or wherein, after mounting the sensor receptacle, the bioreactor is equipped with a sensor, wherein the sensor is introduced at least partially into the sensor receiving area of the holder body.

15. The method according to claim 13 or 14, wherein the sensor receptacle accommodates at least one sensor; and wherein during an operating state the radiation intensity and/or wavelength of the electromagnetic radiation is measured in the interior of the bioreactor, in particular in the interior of the container for holding fluid media containing biological material, in particular in a spatially resolved manner; and wherein in particular electromagnetic radiation of a defined wavelength, preferably 250 nm, is irradiated into the bioreactor for a predefined period of time; and after this irradiation, a radiation intensity and/or wavelength of the electromagnetic radiation is measured in the interior of the bioreactor over a broad range of wavelengths or selectively at one wavelength, in particular at 270 nm.

16. The method according to any one of claims 13 to 15, wherein a sensor (8) is exchanged during the cultivation of biological material in the container (3) of the bioreactor (1), wherein the sterility conditions within the container (3) of the bioreactor (1) are maintained during a replacement or exchange of the sensor (8).

17. The method according to any one of claims 13 to 16, which uses photo- or mixotrophic microorganisms modified by mutagenesis, in particular also microalgae, yeasts, and bacteria.

## Revendications

1. Bioréacteur comprenant
un récipient destiné à recevoir des milieux fluides qui contiennent du matériel biologique,
une voie de passage dotée d'une ouverture de passage entre l'intérieur du récipient destiné à recevoir des milieux fluides qui contiennent du matériel biologique et l'extérieur du récipient destiné à recevoir des milieux fluides qui contiennent du matériel biologique,
**caractérisé en ce**
**qu'**un logement de capteur destiné au maintien d'au moins un capteur s'étend au moins en partie dans l'ouverture de passage de la voie de passage, le logement de capteur obturant avec sa fenêtre l'ouverture de passage
et le logement de capteur comprend un corps de maintien qui comporte une bride standard.

2. Bioréacteur selon la revendication 1, **caractérisé en ce que** le bioréacteur est autoclavable conjointement avec le logement de capteur destiné au maintien d'au moins un capteur, en particulier est autoclavable alors que ce dernier est au moins en partie maintenu dans l'ouverture de passage de la voie de passage.

3. Logement de capteur destiné au maintien d'au moins un capteur pour un bioréacteur, en particulier pour un bioréacteur ayant les caractéristiques de l'une des deux revendications précédentes, **caractérisé par** un corps de maintien comportant une zone de logement de capteur, au niveau de laquelle est disposée une fenêtre comportant un élément transparent qui est apte à la transmission de rayonnement électromagnétique,
le corps de maintien comprenant une bride standard.

4. Logement de capteur pour un bioréacteur selon la revendication 3, **caractérisé en ce que** le corps de maintien est configuré en symétrie cylindrique, en particulier sous forme de colonne, et comporte une ouverture de passage qui est étanche au fluides, en particulier fermée de façon hermétiquement étanche, au moyen de la fenêtre du côté tourné vers l'intérieur du bioréacteur, de préférence l'élément transparent de la fenêtre présentant dans une région spectrale ayant une longueur d'onde de 250 à 2 000 nm une transmission qui est supérieure à 80 %, de façon particulièrement préférée supérieure à 90 %.

5. Logement de capteur pour un bioréacteur selon la revendication 3 ou 4, **caractérisé en ce que** l'élément transparent de la fenêtre comprend du verre ou consiste en verre et
de préférence comprend du verre de quartz ou du verre de borosilicate ou consiste en un tel verre.

6. Logement de capteur pour un bioréacteur selon l'une quelconque des revendications 3 à 5, **caractérisé en ce que** l'élément transparent de la fenêtre est maintenu au moyen d'un vitrage par pression à scellement verre-métal (GTMS) sur un corps de base et ce dernier est maintenu sur le corps de maintien du logement de capteur, et/ou la fenêtre est assemblée de façon hermétiquement étanche avec le corps de maintien par soudage, en particulier soudage laser.

7. Logement de capteur pour un bioréacteur selon l'une quelconque des revendications 3 à 6, **caractérisé en ce que** l'élément transparent de la fenêtre est configuré en forme de disque et en particulier présente des surfaces principales planes et parallèles ou est en forme plano-convexe, plano-concave, biconvexe, biconcave, convexe-concave ou concave-convexe.

8. Logement de capteur pour un bioréacteur selon l'une quelconque des revendications 3 à 7, **caractérisé en ce que** l'élément transparent de la fenêtre fait partie d'un système générateur d'image affecté à celui-ci.

9. Logement de capteur pour un bioréacteur selon l'une quelconque des revendications 3 à 8, **caractérisé en ce que** le corps de maintien comporte un talon latéral s'étendant en direction radiale et de préférence, lorsque le logement de capteur est inséré dans l'ouverture de passage, la distance axiale de l'élément transparent de la fenêtre à la paroi interne du bioréacteur est définie par la distance axiale de l'élément transparent de la fenêtre au talon latéral.

10. Logement de capteur pour un bioréacteur selon l'une quelconque des revendications 3 à 9, **caractérisé en ce que** la bride standard s'étend radialement vers l'extérieur et définit dans la direction axiale une surface d'appui pour un moyen d'étanchéité.

11. Capteur pour un logement de capteur selon l'une quelconque des revendications 3 à 10, **caractérisé en ce que** le capteur comprend au moins une première partie de capteur qui peut être introduite par emboîtement dans l'ouverture de passage du corps de maintien et comprend au moins un dispositif capteur.

12. Capteur pour un logement de capteur selon la revendication 11, **caractérisé en ce que** le dispositif capteur comprend un guide d'image, en particulier un guide d'image à fibres et/ou le dispositif capteur comprend un système optique générateur d'image, en particulier conjointement avec un dispositif récepteur d'image et/ou le dispositif capteur comprend un spectromètre.

13. Procédé pour la multiplication ou la culture de matériel biologique, comprenant l'introduction de fluides, en particulier de matériel biologique ou d'un précurseur de matériel biologique dans un bioréacteur selon la revendication 1 ou 2, l'acquisition d'une grandeur physique, chimique ou biologique mesurée, avec utilisation d'un logement de récepteur ayant les caractéristiques des revendications 3 à 10 ainsi que d'un capteur ayant les caractéristiques de la revendication 11 ou 12,
comprenant en particulier la fabrication de produits pharmaceutiques, en particulier de produits biopharmaceutiques.

14. Procédé pour la multiplication ou la culture selon la revendication 13, dans lequel on stérilise le bioréacteur après la mise en place du logement de capteur, et/ou
on munit d'un capteur le bioréacteur après la mise en place du logement de capteur, en introduisant le capteur au moins en partie dans la zone de logement de capteur du corps de maintien.

15. Procédé selon la revendication 13 ou 14, dans lequel le logement de capteur reçoit au moins un capteur, et dans l'état de fonctionnement l'intensité de rayonnement et/ou la longueur d'onde du rayonnement électromagnétique est/sont mesurée(s), en particulier mesurée(s) en résolution locale, à l'intérieur du bioréacteur, en particulier à l'intérieur du récipient destiné à recevoir des milieux fluides qui contiennent du matériel biologique et en particulier
un rayonnement électromagnétique d'une longueur d'onde définie, de préférence 250 nm, est irradié dans le bioréacteur pendant un intervalle de temps défini et après cette irradiation une intensité de rayonnement et/ou une longueur d'onde du rayonnement électromagnétique est/sont mesurée(s) sur une large bande ou sélectivement à une longueur d'onde, en particulier à 270 nm, à l'intérieur du bioréacteur.

16. Procédé selon l'une quelconque des revendications 13 à 15, dans lequel pendant la culture de matériel biologique dans le récipient (3) du bioréacteur (1) on change un capteur (8),
en maintenant les conditions de stérilité à l'intérieur du récipient (3) du bioréacteur (1) pendant un remplacement ou échange du capteur (8).

17. Procédé selon l'une quelconque des revendications 13 à 16, dans lequel on utilise des micro-organismes, en particulier également des microalgues, levures et bactéries, photo- ou mixotrophes, modifiés par mutagenèse.
